# EUROPÄISCHE PATENTSCHRIFT

(11) **EP 1 641 800 B1**
(45) Veröffentlichungstag und Bekanntmachung des Hinweises auf die Patenterteilung: **05.03.2008**
(21) Anmeldenummer: 04740055.1
(22) Anmeldetag: 18.06.2004
(51) Int. Cl.: C07D 213/61, C07D 213/55, A01N 43/90

(54) **PYRAZOLOPYRIMIDINE**
PYRAZOLOPYRIMIDINES
PYRAZOLOPYRIMIDINES

(30) Priorität: 27.06.2003 DE 10328996; 27.08.2003 DE 10339360; 10.12.2003 DE 10357570
(43) Veröffentlichungstag der Anmeldung: 05.04.2006
(73) Patentinhaber: Bayer CropScience AG, 40789 Monheim (DE)
(72) Erfinder: GEBAUER, Olaf, 51377 Leverkusen (DE); HEINEMANN, Ulrich, 42799 Leichlingen (DE); HERRMANN, Stefan, 40764 Langenfeld (DE); GUTH, Oliver, 51371 Leverkusen (DE); ELBE, Hans-Ludwig, 42329 Wuppertal (DE); GAYER, Herbert, 40789 Monheim (DE); GREUL, Jörg, Nico, 42799 Leichlingen (DE); HILLEBRAND, Stefan, 41462 Neuss (DE); EBBERT, Ronald, 90475 Nürnberg (DE); WACHENDORFF-NEUMANN, Ulrike, 56566 Neuwied (DE); DAHMEN, Peter, 41470 Neuss (DE); KUCK, Karl-Heinz, 40764 Langenfeld (DE)
(86) Internationale Anmeldenummer: PCT/EP2004/006609
(87) Internationale Veröffentlichungsnummer: WO 2005/000851

(56) Entgegenhaltungen:
- US-A- 4 908 056
- NOVINSON T ET AL: "Synthesis and antifungal properties of certain 7-alkylaminopyrazolo(1,5-a)pyrimidines" JOURNAL OF MEDICINAL CHEMISTRY, AMERICAN CHEMICAL SOCIETY, US, Bd. 20, Nr. 2, 1977, Seiten 296-299, XP002970049 ISSN: 0022-2623
- NOVINSON T ET AL: "NOVEL HETEROCYCLIC NITROFURFURAL HYDRAZONES. IN VIVO ANTITRYPANOSOMAL ACTIVITY" JOURNAL OF MEDICINAL CHEMISTRY, AMERICAN CHEMICAL SOCIETY. WASHINGTON, US, Bd. 19, Nr. 4, 1976, Seiten 512-516, XP002030876 ISSN: 0022-2623

## Beschreibung

Die vorliegende Erfindung betrifft neue Pyrazolopyrimidine, mehrere Verfahren zu deren Herstellung und deren Verwendung zur Bekämpfung von unerwünschten Mikroorganismen. Die Erfindung betrifft außerdem neue Zwischenprodukte sowie Verfahren zu ihrer Herstellung.

Es ist bereits bekannt geworden, dass bestimmte Pyrazolopyrimidine fungizide Eigenschaften besitzen (vergleiche DE-A 3 130 633 oder FR-A 2 794 745). Die Wirkung dieser Stoffe ist gut, lässt aber bei niedrigen Aufwandmengen in manchen Fällen zu wünschen übrig.

Es wurden nun neue Pyrazolopyrimidine der Formel 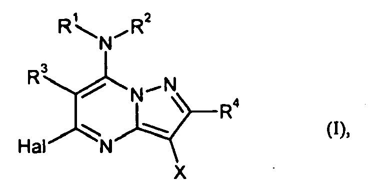 in welcher
- *R¹*: *für Alkyl mit 1 bis 6 Kohlenstoffatomen steht, das einfach bis fünffach, gleichartig oder verschieden substituiert sein kann durch Halogen, Cyano, Hydroxy, Alkoxy mit 1 bis 4 Kohlenstoffatomen und*/*oder Cycloalkyl mit 3 bis 6 Kohlenstoffatomen, oder*
- *R¹*: *für Alkenyl mit 2 bis 6 Kohlenstoffatomen steht, das einfach bis dreifach, gleichartig oder verschieden substituiert sein kann durch Halogen, Cyano, Hydroxy, Alkoxy mit 1 bis 4 Kohlenstoffatomen und*/*oder Cycloalkyl mit 3 bis 6 Kohlenstoffatomen, oder*
- *R¹*: *für Alkinyl mit 3 bis 6 Kohlenstoffatomen steht, das einfach bis dreifach, gleichartig oder verschieden substituiert sein kann durch Halogen, Cyano, Alkoxy mit 1 bis 4 Kohlenstoffatomen und*/*oder Cycloalkyl mit 3 bis 6 Kohlenstoffatomen, oder*
- *R¹*: *für Cycloalkyl mit 3 bis 6 Kohlenstoffatomen steht, das einfach bis dreifach, gleichartig oder verschieden substituiert sein kann durch Halogen und*/*oder Alkyl mit 1 bis* 4 *Kohlenstoffatomen, oder*
- *R¹* R²: *für gesättigtes oder ungesättigtes Heterocyclyl mit 5 oder 6 Ringgliedern und 1 bis 3 Heteroatomen, wie Stickstoff, Sauerstoff und*/*oder Schwefel, stellt, wobei das Heterocyclyl einfach oder zweifach substituiert sein kann durch Halogen, Alkyl mit 1 bis 4 Kohlenstoffatomen, Cyano, Nitro und*/*oder Cycloalkyl mit 3 bis 6 Kohlenstoffatomen,* für Wasserstoff oder Alkyl steht, oder
- R¹ und R²: gemeinsam mit dem Stickstoffatom, an das sie gebunden sind, für einen gesättigten oder ungesättigten heterocyclischen Ring mit 3 bis 6 Ringgliedern stehen, wobei der Heterocyclus ein weiteres Stickstoff-, Sauerstoff- oder Schwefelatom als Ringglied enthalten kann und wobei der Heterocyclus bis zu dreifach substituiert sein kann durch Fluor, Chlor, Brom, Alkyl mit 1 bis 4 Kohlenstoffatomen und/oder Halogenalkyl mit 1 bis 4 Kohlenstoffatomen und 1 bis 9 Fluor- und/oder Chloratomen,
- *R³*: *für gesättigtes oder ungesättigtes Heterocyclyl mit 5 oder 6 Ringgliedern und 1 bis 4 Heteroatomen, wie Sauerstoff, Stickstoff und*/*oder Schwefel, steht, wobei das Heterocyclyl einfach bis vierfach, gleichartig oder verschieden substituiert sein kann durch*
*Fluor, Chlor, Brom, Cyano, Nitro, Alkyl, Alkoxy, Hydroximinoalkyl oder Alkoximinoalkyl mit jeweils 1 bis 3 Kohlenstoffatomen pro Alkylteil,*
*Halogenalkyl oder Halogenalkoxy mit jeweils 1 bis 3 Kohlenstoffatomen und 1 bis 7 Halogenatomen,*
- R⁴: für Wasserstoff oder Alkyl steht,
- Hal: für Halogen steht und
- X: für Halogen, Cyano, Nitro, Alkyl, gegebenenfalls substituiertes Alkenyl, gegebenenfalls substituiertes Alkinyl, Hydroxyalkyl, Alkoxyalkyl, Halogenalkyl, Cycloalkyl, Formyl, Thiocarbamoyl, Alkoxycarbonyl, Alkylcarbonyl, Hydroxyiminoalkyl, Alkoximinoalkyl, Alkylthio, Alkylsulfinyl, Alkylsulfonyl oder Alkylaminocarbonyl steht,
gefunden.

Die erfindungsgemäßen Verbindungen können je nach Substitutionsmuster gegebenenfalls als Mischungen verschiedener möglicher isomerer Formen, insbesondere von Stereoisomeren, wie E-und Z-, threo- und erythro-, sowie optischen Isomeren, gegebenenfalls aber auch in Form von Tautomeren vorliegen. Ist R³ an beiden Atomen, die der Bindungsstelle benachbart sind, ungleich substituiert, können die betreffenden Verbindungen in einer besonderen Form der Stereoisomerie vorliegen, und zwar als Atropisomere.

Weiterhin wurde gefunden, dass sich Pyrazolopyrimidine der Formel (I) herstellen lassen, indem man

### a) Halogen-pyrazolopyrimidine der Formel

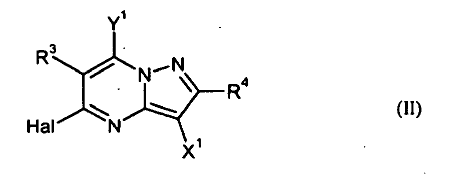

in welcher
- R³, R⁴: und Hal die oben angegebenen Bedeutungen haben,
- X¹: für Halogen, Cyano, Nitro, Alkyl, Halogenalkyl, Cycloalkyl, Formyl, Thiocarbamoyl, Alkoxycarbonyl, Alkylcarbonyl, Alkylthio, Alkylsulfinyl, Alkylsulfonyl oder Alkylaminocarbonyl steht und
- Y¹: für Halogen steht,
mit Aminen der Formel 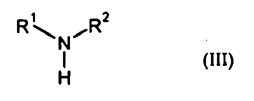 in welcher
- R¹ und R²: die oben angegebenen Bedeutungen haben, gegebenenfalls in Gegenwart eines Verdünnungsmittels, gegebenenfalls in Gegenwart eines Katalysators und gegebenenfalls in Gegenwart eines Säureakzeptors, umsetzt,
oder

### b) Pyrazolopyrimidine der Formel

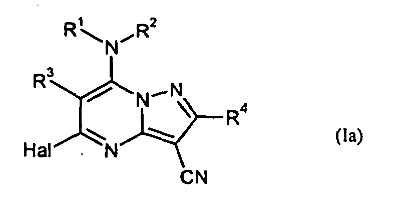

in welcher
R¹, R², R³, R⁴ und Hal die oben angegebenen Bedeutungen haben,
entweder
- α): mit Diisobutyl-aluminiumhydrid in Gegenwart von wässriger Ammoniumchlorid-Lösung sowie in Gegenwart eines organischen Verdünnungsmittels umsetzt,
oder
- β): mit Grignard-Verbindungen der Formel

R⁵ - Mg - X² (IV)

in welcher
R⁵ für Alkyl steht und
X² für Chlor oder Brom steht, in Gegenwart eines Verdünnungsmittels und gegebenenfalls in Gegenwart eines Katalysator umsetzt,
oder

### c) Pyrazolopyrimidine der Formel

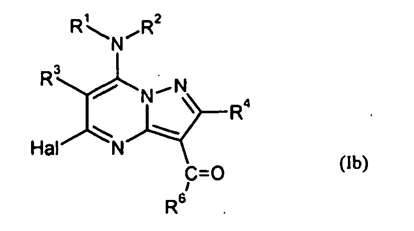

in welcher
- R¹, R², R³, R⁴: und Hal die oben angegebenen Bedeutungen haben und
- R⁶: für Wasserstoff oder Alkyl steht,
entweder
- α): mit Amino-Verbindungen der Formel

H₂N-OR⁷ (V)

in welcher
R⁷ für Wasserstoff oder Alkyl steht, in Gegenwart eines Verdünnungsmittels und gegebenenfalls in Gegenwart eines Katalysators umsetzt, wobei die Amino-Verbindungen der Formel (V) auch in Form ihrer Säureadditions-Salze eingesetzt werden können,
oder
- β): mit Triphenylphosphonium-Salzen der Formel 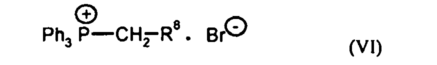 in welcher
Ph für Phenyl steht und
R⁸ für Wasserstoff oder gegebenenfalls substituiertes Alkyl steht,
in Gegenwart einer Base sowie in Gegenwart eines Verdünnungsmittels umsetzt,
oder
- γ): mit Diisobutyl-aluminiumhydrid in Gegenwart von wässriger Ammoniumchlorid-Lösung sowie in Gegenwart eines organischen Verdünnungsmittels umsetzt,
oder mit Natriumborhydrid in Gegenwart eines Verdünnungsmittels umsetzt, und gegebenenfalls die dabei entstehenden Pyrazolopyrimidine der Formel 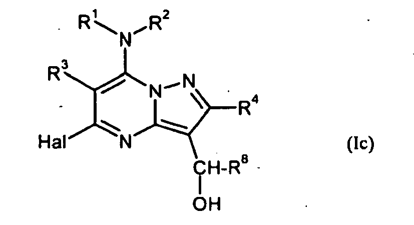 in welcher
R¹, R², R³, R⁴, R⁸ und Hal die oben angegebenen Bedeutungen haben,
mit Alkylierungsmitteln der Formel

R⁹-X³ (VII)

in welcher
- R⁹: für Alkyl steht und
- X³: für Chlor, Brom, Iod oder den Rest R⁹ O-SO₂-O- steht,
gegebenenfalls in Gegenwart einer Base und in Gegenwart eines Verdünnungsmittels umsetzt,
oder

### d) Pyrazolopyrimidine der Formel

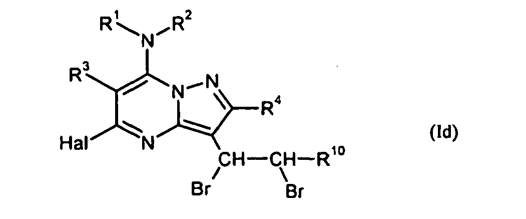

in welcher
R¹, R², R³, R⁴ und Hal die oben angegebenen Bedeutungen haben,
R¹⁰ für Wasserstoff oder gegebenenfalls substituiertes Alkyl steht, mit starken Basen in Gegenwart eines Verdünnungsmittels umsetzt, oder

### e) Pyrazolopyrimidine der Formel

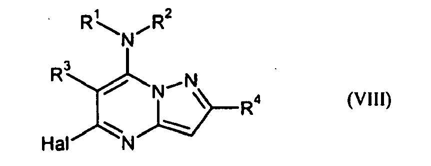

in welcher
R¹, R², R³, R⁴ und Hal die oben angegebenen Bedeutungen haben,
mit Acyl-Derivaten der Formel 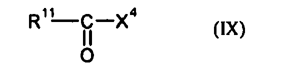 in welcher
- R¹¹: für Alkyl steht und
- X⁴: für Chlor oder einen Rest der Formel 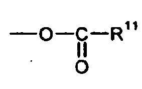 steht,
in Gegenwart eines Katalysators und in Gegenwart eines Verdünnungsmittels umsetzt.

Schließlich wurde gefunden, dass sich die Pyrazolopyrimidine der Formel (I) sehr gut zur Bekämpfung von unerwünschten Mikroorganismen eignen. Sie zeigen vor allem eine starke fungizide Wirksamkeit und lassen sich sowohl im Pflanzenschutz als auch im Materialschutz verwenden.

Überraschenderweise besitzen die erfindungsgemäßen Pyrazolopyrimidine der Formel (I) eine wesentlich bessere mikrobizide Wirksamkeit als die konstitutionell ähnlichsten, vorbekannten Stoffe gleicher Wirkungsrichtung.

Die erfindungsgemäßen Verbindungen der Formel (I) können gegebenenfalls als Mischungen verschiedener möglicher isomerer Formen, insbesondere von Stereoisomeren, wie E- und Z-, threo-und erythro-, sowie optischen Isomeren, wie R- und S-Isomeren oder Atropisomeren, gegebenenfalls aber auch von Tautomeren vorliegen.

Sowohl die reinen Stereoisomeren als auch beliebige Gemische dieser Isomeren sind Gegenstand dieser Erfindung, auch wenn hier im Allgemeinen nur von den Verbindungen der Formel (I) die Rede ist.

Je nach Art der oben definierten Subtituenten weisen die Verbindungen der Formel (I) saure oder basische Eigenschaften auf und können Salze bilden. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄)-Alkylresten sowie Mono-, Di- und Trialkanolamine von (C₁-C₄)-Alkanolen. Tragen die Verbindungen der Formel (I) Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz, Schwefel- und Phosphorsäure, organische Säuren, wie Essigsäure oder Oxalsäure, und saure Salze, wie NaHSO₄ und KHSO₄. Die so erhältlichen Salze weisen ebenfalls fungizide und mikrobizide Eigenschaften auf.

Gegenstand der Erfindung sind auch die aus Verbindungen der Formel (I) durch Umsetzung mit basischen bzw. sauren Verbindungen gebildeten salzartigen Derivate sowie die nach üblichen Oxygenierungsmethoden herstellbaren N-Oxide.

Heterocyclyl steht im vorliegenden Fall für gesättigte oder ungesättigte, aromatische oder nichtaromatische, ringförmige Verbindungen mit 3 bis 8 Ringgliedern, in denen mindestens ein Ringglied ein Heteroatom ist, also ein von Kohlenstoff verschiedenes Atom darstellt. Enthält der Ring mehrere Heteroatome, so können diese gleich oder verschieden sein. Heteroatome sind bevorzugt Sauerstoff, Stickstoff oder Schwefel. Enthält der Ring mehrere Sauerstoffatome, so stehen diese nicht direkt benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono-oder bicyclische, aromatische Ringsysteme.

Die erfindungsgemäßen Pyrazolopyrimidine sind durch die Formel (I) allgemein definiert. Bevorzugt sind diejenigen Stoffe der Formel (I), in denen
- R¹: für Alkyl mit 1 bis 6 Kohlenstoffatomen steht, das einfach bis fünffach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen, oder
- R¹: für Alkenyl mit 2 bis 6 Kohlenstoffatomen steht, das einfach bis dreifach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen, oder
- R¹: für Alkinyl mit 3 bis 6 Kohlenstoffatomen steht, das einfach bis dreifach, gleichartig oder verschieden substituiert sein kann durch Halogen, Cyano, Alkoxy mit 1 bis 4 Kohlenstoffatomen und/oder Cycloalkyl mit 3 bis 6 Kohlenstoffatomen, oder
- R¹: für Cycloalkyl mit 3 bis 6 Kohlenstoffatomen steht, das einfach bis dreifach, gleichartig oder verschieden substituiert sein kann durch Halogen und/oder Alkyl mit 1 bis 4 Kohlenstoffatomen, oder
- R¹: für gesättigtes oder ungesättigtes Heterocyclyl mit 5 oder 6 Ringgliedern und 1 bis 3 Heteroatomen, wie Stickstoff, Sauerstoff und/oder Schwefel, steht, wobei das Heterocyclyl einfach oder zweifach substituiert sein kann durch Halogen, Alkyl mit 1 bis 4 Kohlenstoffatomen, Cyano, Nitro und/oder Cycloalkyl mit 3 bis 6 Kohlenstoffatomen,
- R²: für Wasserstoff oder Alkyl mit 1 bis 4 Kohlenstoffatomen steht, oder
- R¹ und R²: gemeinsam mit dem Stickstoffatom, an das sie gebunden sind, für einen gesättigten oder ungesättigten heterocyclischen Ring mit 3 bis 6 Ringgliedern stehen, wobei der Heterocyclus ein weiteres Stickstoff-, Sauerstoff- oder Schwefelatom als Ringglied enthalten kann und wobei der Heterocyclus bis zu dreifach substituiert sein kann durch Fluor, Chlor, Brom, Alkyl mit 1 bis 4 Kohlenstoffatomen und/oder Halogenalkyl mit 1 bis 4 Kohlenstoffatomen und 1 bis 9 Fluor- und/oder Chloratomen,
- R³: für gesättigtes oder ungesättigtes Heterocyclyl mit 5 oder 6 Ringgliedern und 1 bis 4 Heteroatomen, wie Sauerstoff, Stickstoff und/oder Schwefel, steht, wobei das Heterocyclyl einfach bis vierfach, gleichartig oder verschieden substituiert sein kann durch
Fluor, Chlor, Brom, Cyano, Nitro, Alkyl, Alkoxy, Hydroximinoalkyl oder Alkoximinoalkyl mit jeweils 1 bis 3 Kohlenstoffatomen pro Alkylteil,
Halogenalkyl oder Halogenalkoxy mit jeweils I bis 3 Kohlenstoffatomen und 1 bis 7 Halogenatomen,
- R⁴: für Wasserstoff oder Alkyl mit 1 bis 4 Kohlenstoffatomen steht,
- Hal: für Fluor, Chlor oder Brom steht und
- X: für Cyano, Fluor, Chlor, Brom, Iod, Nitro, Formyl, Halogenalkyl mit 1 bis 6 Kohlenstoffatomen und 1 bis 9 Fluor, Chlor und/oder Bromatomen, Alkyl mit 1 bis 4 Kohlenstoffatomen, Alkenyl mit 2 bis 6 Kohlenstoffatomen, durch Carboxy, Methoxycarbonyl oder Ethoxycarbonyl substituiertes Alkenyl mit 2 bis 5 Kohlenstoffatomen im Alkenylteil, Alkinyl mit 2 bis 6 Kohlenstoffatomen, durch Carboxy, Methoxycarbonyl oder Ethoxycarbonyl substituiertes Alkinyl mit 2 bis 5 Kohlenstoffatomen, Hydroxyalkyl mit 1 bis 4 Kohlenstoffatomen, Alkoxyalkyl mit 1 bis 4 Kohlenstoffatomen im Alkoxyteil und 1 bis 4 Kohlenstoffatomen im Alkylteil, Cycloalkyl mit 3 bis 6 Kohlenstoffatomen, Thiocarbamoyl, Alkoxycarbonyl mit 1 bis 4 Kohlenstoffatomen im Alkoxyteil, Alkylcarbonyl mit 1 bis 4 Kohlenstoffatomen im Alkylteil, Hydroximinoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen oder für Alkylaminocarbonyl mit 1 bis 4 Kohlenstoffatomen im Alkylteil steht.

Besonders bevorzugt sind diejenigen Pyrazolopyrimidine der Formel (I), in denen
- R¹: für einen Rest der Formel 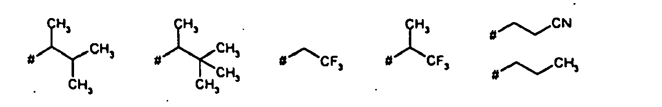 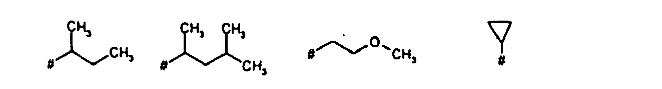 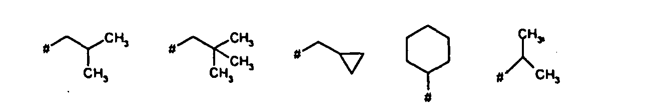 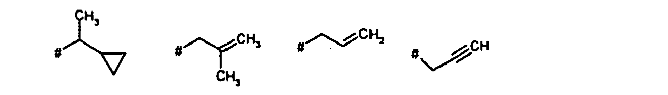 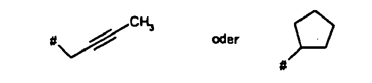 steht.
wobei # die Anknüpfungsstelle markiert,
- R²: für Wasserstoff, Methyl, Ethyl oder Propyl steht, oder
- R¹ und R²: gemeinsam mit dem Stickstoffatom, an das sie gebunden sind, für Pyrrolidinyl, Piperidinyl, Morpholinyl, Thiomorpholinyl, Piperazinyl, 3,6-Dihydro-1(2H)-piperidinyl öder Tetrahydro-1(2H)-pyridazinyl stehen, wobei diese Reste durch 1 bis 3 Fluoratome, 1 bis 3 Methylgruppen und/oder Trifluormethyl substituiert sein können,
oder
- R¹ und R²: gemeinsam mit dem Stickstoffatom, an das sie gebunden sind, für einen Rest der Formel 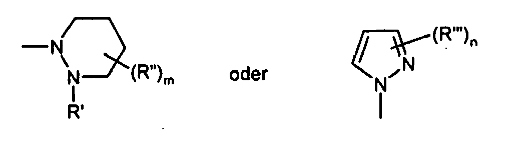
worin
- R': für Wasserstoff oder Methyl steht,
- R": für Methyl, Ethyl, Fluor, Chlor oder Trifluormethyl steht,
- m: für die Zahlen 0, 1, 2 oder 3 steht, wobei R" fUr gleiche oder verschiedene Reste steht, wenn m für 2 oder 3 steht,
- R"': für Methyl, Ethyl, Fluor, Chlor oder Trifluormethyl steht
und
- n: für die Zahlen 0, 1, 2 oder 3 steht, wobei R'" für gleiche oder verschiedene Reste steht, wenn n für 2 oder 3 steht,
- R³: für Pyridyl steht, das in 2- oder 4-Stellung verknüpft ist und einfach bis vierfach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinomethyl, Methoximinoethyl und/oder Trifluormethyl, oder
- R³: für Pyrimidyl steht, das in 2- oder 4-Stellung verknüpft ist und einfach bis dreifach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinomethyl, Methoximinoethyl und/oder Trifluormethyl, oder
- R³: für Thienyl steht, das in 2- oder 3-Stellung verknüpft ist und einfach bis dreifach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinimethyl, Methoximinoethyl und/oder Trifluormethyl, oder
- R³: für Thiazolyl steht, das in 2-, 4- oder 5-Stellung verknüpft ist und einfach bis zweifach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinimethyl, Methoximinoethyl und/oder Trifluormethyl,
- R⁴: für Wasserstoff, Methyl, Ethyl, Propyl oder Isopropyl steht,
- Hal: für Fluor oder Chlor steht und
- X: für Cyano, Fluor, Chlor, Brom, Jod, Nitro, Formyl, Trifluormethyl, Difluormethyl, Methyl, Ethyl, Cyclopropyl, Thiocarbamoyl, Methoxycarbonyl, Methylcarbonyl, Ethylcarbonyl, Hydroximinomethyl, Methoximinomethyl, Methylthio, Methylsulfinyl Methylsulfonyl, Methylaminocarbonyl, Hydroxymethyl, Hydroxyeth-1-yl, Methoxymethyl, Ethoxymethyl oder 1-Methoxy-ethyl steht, oder
- X: für einen Rest der Formel

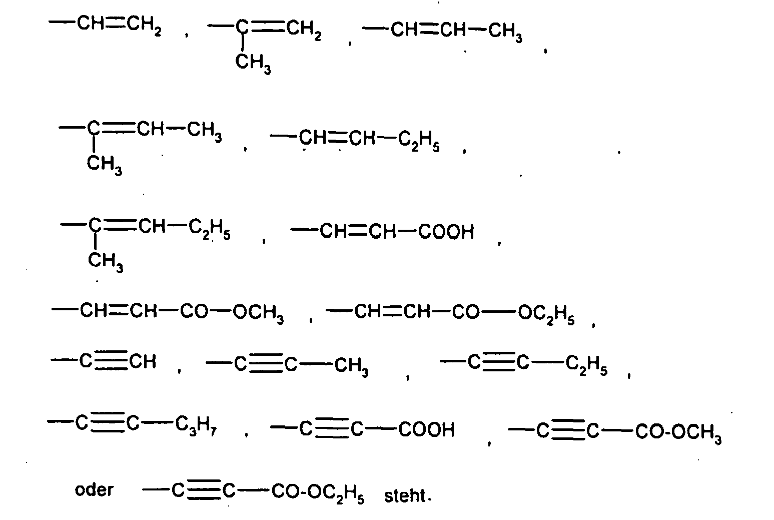

Die zuvor genannten Reste-Definitionen können untereinander in beliebiger Weise kombiniert werden. Außerdem können einzelne Definitionen entfallen.

Verwendet man 3-Cyano-5,7-dichlor-6-(3-trifluormethyl-pyridin-2-yl)-pyrazolo[1,5-a]pyrimidin und 2,2,2-Trifluorisopropylamin als Ausgangsstoffe, so kann der Verlauf des erfindungsgemäßen Verfahrens (a) durch das folgende Formelschema veranschaulicht werden.

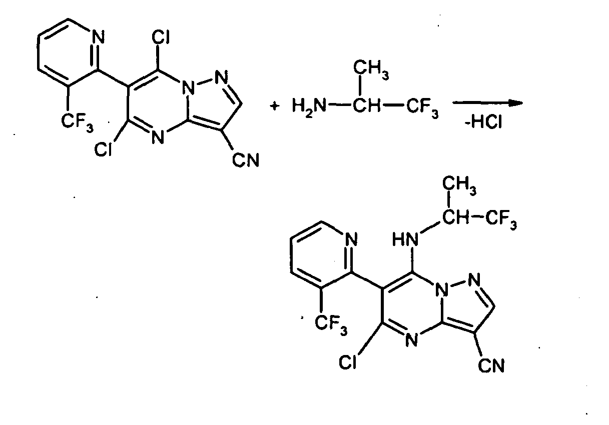

Verwendet man 3-Cyano-5-chlor-6-(3-trifluormethyl-pyridin-2-yl)-7-(2,2,2-trifluorisopropyl-amino)-pyrazolo[1,5-a]pyrimidin als Ausgangsstoff und Di-isobutyl-aluminiumhydrid als Reaktionskomponente, so kann der Verlauf des erfindungsgemäßen Verfahrens (b, Variante α) durch das folgende Formelschema veranschaulicht werden.

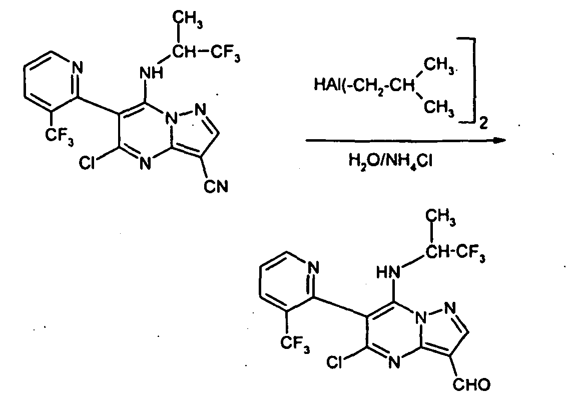

Verwendet man 3-Cyano-5-chlor-6-(3-trifluormethyl-pyridin-2-yl)-7-(2,2,2-trifluorisopropyl-amino)-pyrazolo[1,5-a]pyrimidin als Ausgangsstoff und Methyl-magnesium-bromid als Reaktionskomponente, so kann der Verlauf des erfindungsgemäßen Verfahrens (b, Variante β) durch das folgende Formelschema veranschaulicht werden.

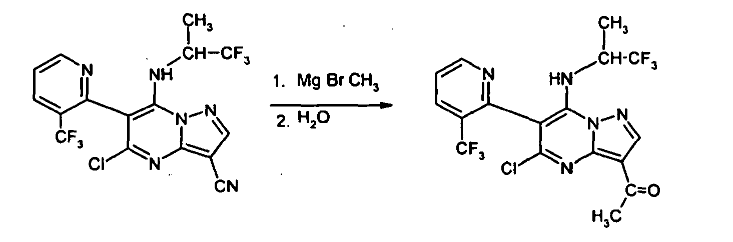

Verwendet man 3-Formyl-5-chlor-6-(3-trifluormethyl-pyridin-2-yl)-7-(2,2,2-trifluorisopropyl-amino)-pyrazolo-[1,5a]pyrimidin und Methoxyamin-hydrochlorid als Ausgangsstoffe, so kann der Verlauf des erfindungsgemäßen Verfahrens (c, Variante α) durch das folgende Formelschema veranschaulicht werden.

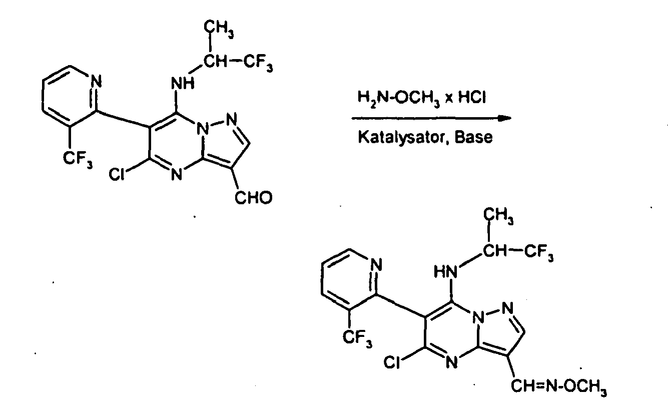

Verwendet man 3-Methylcarbonyl-5-chlor-6-(3-trifluormethyl-pyridin-2-yl)-7-(2,2,2-trifluorisopropylamino)-pyrazolo-[1,5a]pyrimidin als Ausgangsstoff und Triphenyl-methyl-phosphonium-bromid als Reaktionskomponente, so kann der Verlauf des erfindungsgemäßen Verfahrens (c, Variante β) durch das folgende Formelschema veranschaulicht werden.

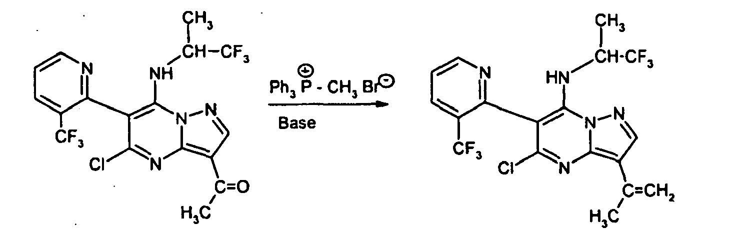

Verwendet man 3-Methylcarbonyl-5-chlor-6-(3-trifluormethyl-pyridin-2-yl)-7-(2,2,2-trifluorisopropylamino)-pyrazolo-[1,5a]pyrimidin als Ausgangsstoff, Diisobutyl-aluminiumhydrid als Reaktionskomponente in der ersten Stufe und Methyliodid als Reaktionskomponente in der zweiten Stufe, so kann der Verlauf des erfindungsgemäßen Verfahrens (c, Variante γ) durch das folgende Formelschema veranschaulicht werden.

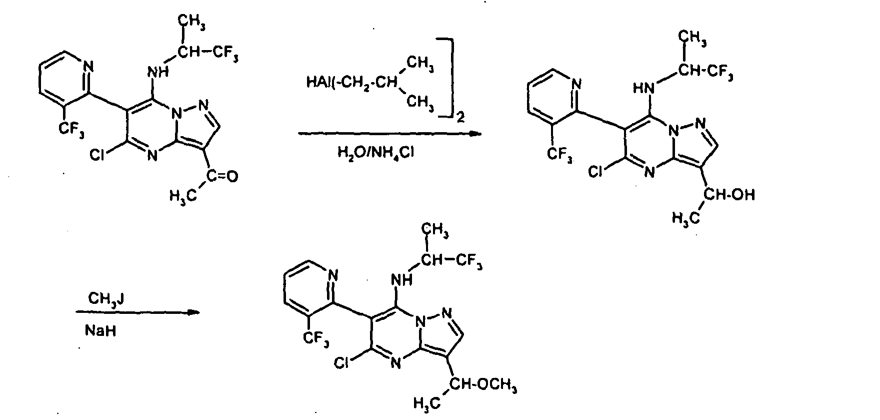

Verwendet man 3-(1,2-Dibrompropyl)-5-chlor-6-(5-chlor-pyrimidin-4-yl)-7-(4-methyl-piperidino)-pyrazolo-[1,5a]pyrimidin als Ausgangsstoff und Kalium-tert.-butylat, als Reaktionskomponente, so kann der Verlauf des erfindungsgemäßen Verfahrens (d) durch das folgende Formelschema veranschaulicht werden.

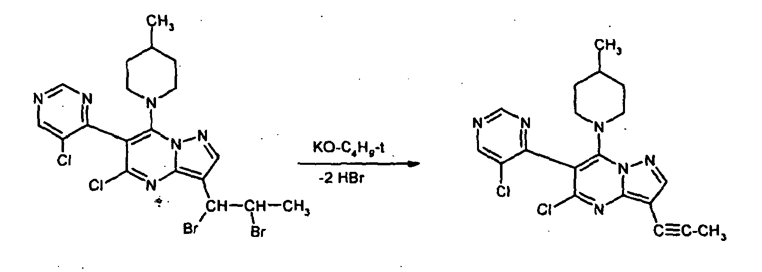

Verwendet man 5-Chlor-6-(5-chlor-pyrimidin-4-yl)-7-(4-methyl-piperidino)-pyrazolo-[1,5a]-pyrimidin als Ausgangsstoff, Acetylchlorid als Reaktionskomponente und Aluminiumtrichlorid als Katalysator, so kann der Verlauf des erfindungsgemäßen Verfahrens (e) durch das folgende Formelschema veranschaulicht werden.

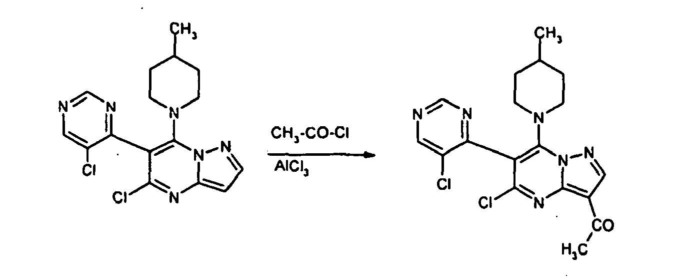

Die zur Durchführung des erfindungsgemäßen Verfahrens (a) als Ausgangsstoffe benötigten Halogenpyrazolopyrimidine sind durch die Formel (II) allgemein definiert. In dieser Formel (II) haben R³, R⁴ und Hal vorzugsweise diejenigen Bedeutungen, die bereits im Zusammenhang mit der Beschreibung der erfindungsgemäßen Verbindungen der Formel (I) für diese Reste als bevorzugt genannt wurden.
- Y¹: steht vorzugsweise für Fluor, Chlor oder Brom, besonders bevorzugt für Fluor oder Chlor.
- X¹: steht vorzugsweise für Cyano, Fluor, Chlor, Brom, Iod, Nitro, Halogenalkyl mit 1 bis 6 Kohlenstoffatomen und 1 bis 9 Fluor-, Chlor- und/oder Bromatomen, Alkyl mit 1 bis 4 Kohlenstoffatomen, Formyl, Cycloalkyl mit 3 bis 6 Kohlenstoffatomen, Thiocarbamoyl, Alkoxycarbonyl mit 1 bis 4 Kohlenstoffatomen im Alkoxyteil, Alkylcarbonyl mit 1 bis 4 Kohlenstoffatomen im Alkylteil, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen oder für Alkylaminocarbonyl mit 1 bis 4 Kohlenstoffatomen im Alkylteil.
- X¹: steht besonders bevorzugt für Cyano, Fluor, Chlor, Brom, Jod, Nitro, Trifluormethyl, Difluormethyl, Methyl, Ethyl, Formyl, Cyclopropyl, Thiocarbamoyl, Methoxycarbonyl, Methylcarbonyl, Methylthio, Ethylcarbonyl, Methylsulfinyl, Methylsulfonyl, Methylaminocarbonyl, 1,2-Dibrompropyl oder 1,2-Dibrom-butyl.

Die Halogen-pyrazolopyrimidine der Formel (II) sind neu. Auch diese Stoffe eignen sich zur Bekämpfung von unerwünschten Mikroorganismen.

Die Halogen-pyrazolopyrimidine der Formel (II) lassen sich herstellen,
indem man

### f) Hydroxy-pyrazolopyrimidine der Formel

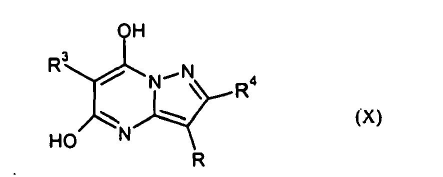

in welcher
- R³ und R⁴: die oben angegebenen Bedeutungen haben, und
- R: für Halogen, Cyano, Nitro, Alkyl, Halogenalkyl, Cycloalkyl, Thiocarbamoyl, Alkoxycarbonyl, Alkylthio, Alkylsulfinyl, Alkylsulfonyl oder Alkylaminocarbonyl steht,
mit Halogenierungsmitteln, gegebenenfalls in Gegenwart eines Verdünnungsmittels umsetzt,
oder

### g) Hydroxy-pyrazolopyrimidine der Formel

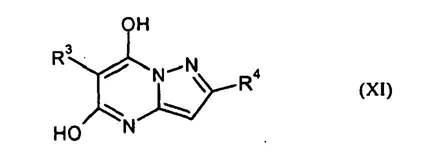

in welcher
R³ und R⁴ die oben angegebenen Bedeutungen haben,
mit Phosphoroxychlorid in Gegenwart von Dimethylformamid umsetzt und gegebenenfalls unter Zugabe von Phosphorpentachlorid nachreagieren lässt.

Verwendet man 3-Cyano-6-(3-trifluormethyl-pyridin-2-yl)-pyrazolo[1,5-a]pyrimidin-5,7-diol als Ausgangsstoff und Phosphoroxychlorid im Gemisch mit Phosphorpentachlorid als Halogenierungsmittel, so kann der Verlauf des erfindungsgemäßen Verfahrens (f) durch das folgende Formelschema veranschaulicht werden.

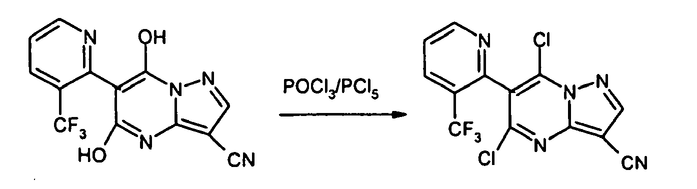

Die zur Durchführung des erfindungsgemäßen Verfahrens (f) als Ausgangsstoffe benötigten Hydroxy-pyrazolopyrimidine sind durch die Formel (X) allgemein definiert. In dieser Formel haben R³ und R⁴ vorzugsweise diejenigen Bedeutungen, die bereits im Zusammenhang mit der Beschreibung der erfindungsgemäßen Verbindungen der Formeln (I) für diese Reste als bevorzugt genannt wurden. R steht vorzugsweise für Cyano, Fluor, Chlor, Brom, Iod, Nitro, Alkyl mit 1 bis 4 Kohlenstoffatomen, Halogenalkyl mit 1 bis 4 Kohlenstoffatomen und 1 bis 9 Fluor-, Chlor-und/oder Bromatomen, Cycloalkyl mit 3 bis 6 Kohlenstoffatomen, Thiocarbamoyl, Alkoxycarbonyl mit 1 bis 4 Kohlenstoffatomen im Alkoxyteil, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen oder für Alkylaminocarbonyl mit 1 bis 4 Kohlenstoffatomen im Alkylteil.
- R: steht besonders bevorzugt für Cyano, Fluor, Chlor, Brom, Iod, Nitro, Trifluormethyl, Difluormethyl, Chlormethyl, Methyl, Ethyl, Cyclopropyl, Thiocarbamoyl, Methoxycarbonyl, Methylthio, Methylsulfinyl, Methylsulfonyl oder Methylaminocarbonyl.

Auch die Hydroxy-pyrazolopyrimidine der Formel (X) sind bisher noch nicht bekannt. Sie lassen sich herstellen, indem man

### (h) Heterocyclylmalonester der Formel

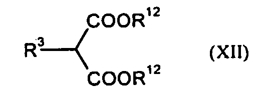

in welcher
- R³: die oben angegebene Bedeutung hat und
- R¹²: für Alkyl mit 1 bis 4 Kohlenstoffatomen steht,
mit Aminopyrazolen der Formel 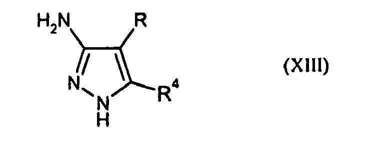 in welcher
- R⁴ und R: die oben angegebenen Bedeutungen haben,
gegebenenfalls in Gegenwart eines Verdünnungsmittels und gegebenenfalls in Gegenwart eines Säurebindemittels umsetzt.

Verwendet man 2-(3-Trifluorrnethyl-pyridin-2-yl)-malonsäuredimethylester und 3-Amino-4-cyano-pyrazol als Ausgangsstoffe, so kann der Verlauf des erfindungsgemäßen Verfahrens (h) durch das folgende Formelschema veranschaulicht werden.

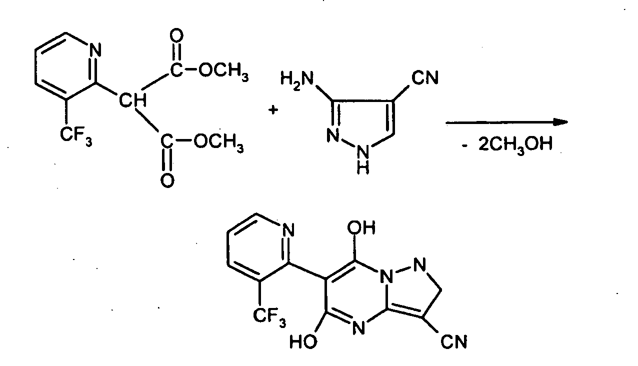

Die zur Durchführung des erfindungsgemäßen Verfahrens (h) als Ausgangsstoffe benötigten Heterocyclylmalonester sind durch die Formel (XII) allgemein definiert. In dieser Formel hat R³ vorzugsweise diejenigen Bedeutungen, die bereits im Zusammenhang mit der Beschreibung der erfindungsgemäßen Stoffe der Formel (I) für diesen Rest als bevorzugt genannt wurden. R¹² steht vorzugsweise für Methyl oder Ethyl.

Die Heterocyclylmalonester der Formel (XII) sind teilweise bekannt (vgl. DE 38 20 538-A, WO O1-11 965 und WO 99-32 464).

Neu sind Pyridylmalonester der Formel 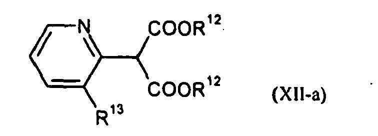 in welcher
- R¹²: die oben angegebene Bedeutung hat und
- R¹³: für Halogen oder Halogenalkyl steht.

Neu sind auch Pyrimidylmalonester der Formel 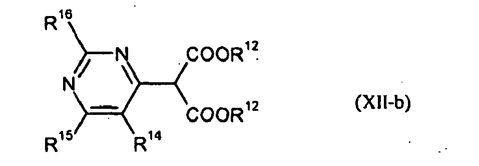 in welcher
- R¹²: die oben angegebene Bedeutung hat,
- R¹⁴: für Halogen oder Halogenalkyl steht, und
- R¹⁵ und R¹⁶: unabhängig voneinander für Wasserstoff, Fluor, Chlor, Brom, Methyl, Ethyl oder Methoxy stehen.

Die Pyridylmalonester der Formel (XII-a) lassen sich herstellen, indem man

### (i) Halogenpyridine der Formel

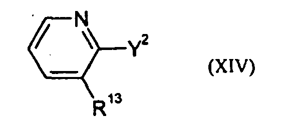

in welcher
- R ¹³: die oben angegebene Bedeutung hat und
- Y²: für Halogen steht,
mit Malonestern der Formel 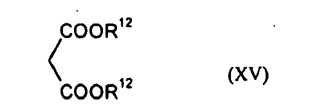 in welcher
- R¹²: die oben angegebene Bedeutung hat,
gegebenenfalls in Gegenwart eines Verdünnungsmittels, gegebenenfalls in Gegenwart eines Kupfersalzes und gegebenenfalls in Gegenwart eines Säureakzeptors umsetzt.

Verwendet man 2-Chlor-3-trifluormethylpyridin und Malonsäuredimethylester als Ausgangsstoffe, so kann der Verlauf des erfindungsgemäßen Verfahrens (i) durch das folgende Formelschema veranschaulicht werden.

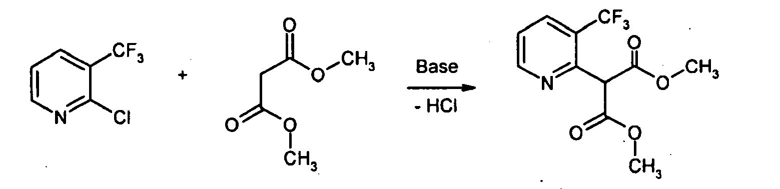

Die zur Durchführung des erfindungsgemäßen Verfahrens (i) als Ausgangsstoffe benötigten Halogenpyridine sind durch die Formel (XIV) allgemein definiert. In dieser Formel steht R¹³ vorzugsweise für Fluor, Chlor oder Trifluormethyl. Y² steht vorzugsweise für Chlor oder Brom.

Die Halogenpyridine der Formel (XIV) sind bekannte Synthesechemikalien.

Die zur Durchführung des erfindungsgemäßen Verfahrens (i) weiterhin als Ausgangsstoffe benötigten Malonsäureester der Formel (XV) sind ebenfalls bekannte Synthesechemikalien.

Die Pyrimidylmalonester der Formel (XII-b) lassen sich herstellen, indem man

### (j) Halogenpyrimidine der Formel

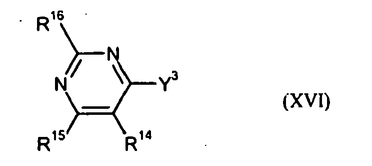

in welcher
- R¹⁴, R¹⁵ und R¹⁶: die oben angegebenen Bedeutungen haben und
- Y³: für Halogen steht,
mit Malonestern der Formel 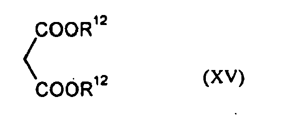 in welcher
- R¹²: die oben angegebene Bedeutung hat,
gegebenenfalls in Gegenwart eines Verdünnungsmittels, gegebenenfalls in Gegenwart eines Kupfersalzes und gegebenenfalls in Gegenwart eines Säureakzeptors umsetzt.

Verwendet man 4,5-Dichlorpyrimidin und Malonsäuredimethylester als Ausgangsstoffe, so kann der Verlauf des erfindungsgemäßen Verfahrens (j) durch das folgende Formelschema veranschaulicht werden.

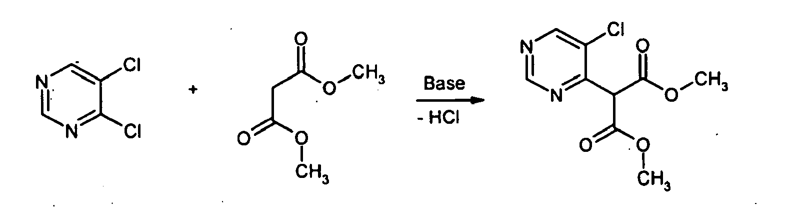

Die zur Durchführung des erfindungsgemäßen Verfahrens (j) als Ausgangsstoffe benötigten Halogenpyrimidine sind durch die Formel (XVI) allgemein definiert. In dieser Formel steht R¹⁴ vorzugsweise für Fluor, Chlor oder Trifluormethyl. R¹⁵ und R¹⁶ stehen auch bevorzugt unabhängig voneinander für Wasserstoff, Fluor, Chlor, Brom, Methyl, Ethyl oder Methoxy. Y³ steht vorzugsweise für Chlor oder Brom.

Die Halogenpyrimidine der Formel (XVI) sind bekannt und können nach bekannten Methoden hergestellt werden (vgl. J. Chem. Soc. 1955, 3478, 3481).

Die zur Durchführung des erfindungsgemäßen Verfahrens (h) als Reaktionskomponten benötigten Aminopyrazole sind durch die Formel (XIII) allgemein definiert. In dieser Formel hat R⁴ vorzugsweise diejenigen Bedeutungen, die bereits im Zusammenhang mit der Beschreibung der erfindungsgemäßen Stoffe der Formel (I) für diesen Rest als bevorzugt genannt wurden. R hat vorzugsweise diejenigen Bedeutungen, die bereits im Zusammenhang mit der Beschreibung der Hydroxy-pyrazolopyrimidine der Formel (X) für diesen Rest als bevorzugt genannt wurden.

Die Aminopyrazole der Formel (XIII) sind bekannt oder können nach bekannten Methoden hergestellt werden.

Als Halogenierungsmittel kommen bei der Durchführung des Verfahrens (f) alle für den Ersatz von Hydroxygruppen durch Halogen üblichen Komponenten in Betracht. Vorzugsweise verwendbar sind Phosphortrichlorid, Phosphortribromid, Phosphorpentachlorid, Phosphoroxychlorid, Thionylchlorid, Thionylbromid oder deren Gemische. Die entsprechenden Fluor-Verbindungen der Formel (II) lassen sich aus den Chlor- oder Brom-Verbindungen durch Umsetzung mit Kaliumfluorid herstellen,

Die genannten Halogenierungsmittel sind bekannt.

Das Verfahren (g) eignet sich zur Herstellung von Halogen-pyrazolopyrimidinen der Formel 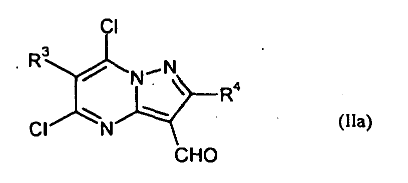 in welcher
- R³ und R⁴: die oben angegebenen Bedeutungen haben.

Die bei der Durchführung des Verfahrens (g) als Ausgangsstoffe benötigten Hydroxy-pyrazolopyrimidine sind durch die Formel (XI) allgemein definiert. In dieser Formel haben R³ und R⁴ vorzugsweise diejenigen Bedeutungen, die bereits im Zusammenhang mit der Beschreibung der erfindungsgemäßen Stoffe der Formel (1) für diese Reste als bevorzugt angegeben wurden.

Die Hydroxy-pyrazolopyrimidine der Formel (XI) lassen sich nach dem Verfahren (h) herstellen, indem man Aminopyrazole der Formel (XIII) einsetzt, in denen R für Wasserstoff steht.

Das Verfahren (g) wird unter den Bedingungen der Vilsmeier-Formylierung mit Hilfe von Phosphoroxychlorid in Gegenwart von Dimethylformamid durchgeführt. Dabei kann auch Phosphorpentachlorid als Chlorierungsmittel hinzugefügt werden.

Die Reaktionstemperaturen können bei der Durchführung des Verfahren (g) in einem größeren Bereich variiert werden. Im Allgemeinen arbeitet man bei Temperaturen zwischen -10°C und +150°C, vorzugsweise zwischen 0°C und 120°C.

Bei der Durchführung des Verfahrens (g) setzt man auf 1 mol an Hydroxy-pyrazolopyrimidinen der Formel (XI) im Allgemeinen 2 bis 5 mol an Dimethylformamid, 5 bis 15 mol Phosphoroxychlorid und gegebenenfalls 0 bis 2 mol Phosphorpentachlorid ein. Die Aufarbeitung erfolgt nach üblichen Methoden.

Die weiterhin zur Durchführung des erfindungsgemäßen Verfahrens (a) als Ausgangsstoffe benötigten Amine sind durch die Formel (III) allgemein definiert. In dieser Formel haben R¹ und

R² vorzugsweise diejenigen Bedeutungen, die bereits im Zusammenhang mit der Beschreibung der erfindungsgemäßen Verbindungen der Formel (I) für R¹ und R² als bevorzugt angegeben wurden.

Die Amine der Formel (III) sind bekannt oder lassen sich nach bekannten Methoden herstellen.

Als Verdünnungsmittel kommen bei der Durchführung des erfindungsgemäßen Verfahrens (a) alle üblichen inerten organischen Solventien in Betracht. Vorzugsweise verwendbar sind halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexaniethylphosphorsäuretriamid; Ester wie Essigsäuremethylester oder Essigsäureethylester; Sulfoxide, wie Dimethylsulfoxid; Sulfone, wie Sulfolan.

Als Säureakzeptoren kommen bei der Durchführung des erfindungsgemäßen Verfahrens (a) alle für derartige Umsetzungen üblichen anorganischen oder organischen Basen in Frage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithium-diisopropylamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat, und außerdem Ammonium Verbindungen wie Ammoniumhydroxid, Ammoniumacetat und Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Katalysatoren kommen bei der Durchführung des erfindungsgemäßen Verfahrens (a) alle für derartige Umsetzungen üblichen Reaktionsbeschleuniger in Betracht. Vorzugsweise verwendbar sind Fluoride wie Natriumfluorid, Kaliumfluorid oder Ammoniumfluorid.

Die Reaktionstemperaturen können bei der Durchführung des erfindungsgemäßen Verfahrens (a) in einem größeren Bereich variiert werden. Im Allgemeinen arbeitet man bei Temperaturen zwischen 0°C und 150°C, vorzugsweise bei Temperaturen zwischen 0°C und 80°C.

Bei der Durchführung des erfindungsgemäßen Verfahrens (a) setzt man auf 1 mol an Halogenpyrazolo-pyrimidin der Formel (II) im Allgemeinen 0,5 bis 10 mol, vorzugsweise 0,8 bis 2 mol an Amin der Formel (III) ein. Die Aufarbeitung erfolgt nach üblichen Methoden.

Die bei der Durchführung des erfindungsgemäßen Verfahrens (b) als Ausgangsstoffe benötigten Pyrazolopyrimidine sind durch die Formel (Ia) allgemein definiert. In dieser Formel haben R¹, R², R³, R⁴ und Hal vorzugsweise diejenigen Bedeutungen, die bereits im Zusammenhang mit der Beschreibung der erfindungsgemäßen Stoffe der Formel (I) für diese Reste als bevorzugt genannt wurden.

Bei den Pyrazolopyrimidinen der Formel (Ia) handelt es sich um erfindungsgemäße Stoffe, die sich nach dem erfindungsgemäßen Verfahren (a) herstellen lassen.

Als Verdünnungsmittel kommen bei der Durchführung des erfindungsgemäßen Verfahrens (b, Variante α) alle üblichen inerten, organischen Solventien in Frage. Vorzugsweise verwendbar sind aliphatische oder aromatische, gegebenenfalls halogenierte Kohlenwasserstoffe, wie Toluol, Dichlormethan, Chloroform oder Tetrachlorkohlenstoff.

Die Reaktionstemperaturen können bei der Durchführung des erfindungsgemäßen Verfahrens (b, Variante α) innerhalb eines bestimmten Bereiches variiert werden. Im Allgemeinen arbeitet man bei Temperaturen zwischen -80°C und +20°C, vorzugsweise zwischen -60°C und +10°C.

Bei der Durchführung des erfindungsgemäßen Verfahrens (b, Variante α) setzt man auf 1 mol an Pyrazolopyrimidin der Formel (Ia) im Allgemeinen eine äquivalente Menge oder auch einen Überschuss, vorzugsweise 1,1 bis 1,2 mol an Di-isobutyl-aluminiumhydrid ein und fügt anschließend einen Überschuss an wässriger Ammoniumchlorid-Lösung hinzu. Die Aufarbeitung erfolgt nach üblichen Methoden. Im Allgemeinen verfährt man in der Weise, dass man das Reaktionsgemisch ansäuert, die organische Phase abtrennt, die wässrige Phase mit einem mit Wasser wenig mischbaren organischen Solvens extrahiert, die vereinigten organischen Phasen wäscht, trocknet und unter vermindertem Druck einengt.

Die bei der Durchführung des erfindungsgemäßen Verfahrens (b, Variante β) als Reaktionskomponenten benötigten Grignard-Verbindungen sind durch die Formel (IV) allgemein definiert. In dieser Formel steht R⁵ vorzugsweise für Alkyl mit 1 bis 4 Kohlenstoffatomen, besonders bevorzugt für Methyl oder Ethyl. X² steht vorzugsweise für Brom.

Als Katalysatoren kommen bei der Durchführung des erfindungsgemäßen Verfahrens (b, Variante β) alle für derartige Grignard-Reaktionen üblichen Reaktionsbeschleuniger in Betracht. Beispielsweise genannt seien Kaliumiodid und Iod.

Als Verdünnungsmittel kommen bei der Durchführung der erfindungsgemäßen Verfahrens (b, Variante β) alle für derartige Umsetzungen üblichen, inerten organischen Solventien in Frage. Vorzugsweise verwendbar sind Ether, wie Diethylether, Dioxan oder Tetrahydrofuran, außerdem aromatische Kohlenwasserstoffe, wie Toluol, und auch Gemische aus Ethern und aromatischen Kohlenwasserstoffen, wie Toluol/Tetrahydrofuran.

Die Reaktionstemperaturen können bei der Durchführung des erfindungsgemäßen Verfahrens (b, Variante β) in einem bestimmten Bereich variiert werden. Im Allgemeinen arbeitet man bei Temperaturen zwischen -20°C und +100°C, vorzugsweise zwischen 0°C und 80°C.

Bei der Durchführung des erfindungsgemäßen Verfahrens (b, Variante β) setzt man auf 1 mol an Pyrazolopyrimidin der Formel (Ia) im Allgemeinen 2 bis 3 mol an Grignard-Verbindung der Formel (IV) ein. Anschließend wird eine wässrige Aufarbeitung nach üblichen Methoden durchgeführt.

Die bei der Durchführung des erfindungsgemäßen Verfahrens (c) als Ausgangsstoffe benötigten Pyrazolopyrimidine sind durch die Formel (Ib) allgemein definiert. In dieser Formel haben R¹, R², R³, R⁴ und Hal vorzugsweise diejenigen Bedeutungen, die bereits im Zusammenhang mit der Beschreibung der erfindungsgemäßen Stoffe der Formel (1) für diese Reste als bevorzugt genannt wurden. R⁶ steht vorzugsweise für Wasserstoff oder Alkyl mit I bis 4 Kohlenstoffatomen, besonders bevorzugt für Wasserstoff, Methyl oder Ethyl.

Bei den Pyrazolopyrimidinen der Formel (1b) handelt es sich um erfindungsgemäße Stoffe, die sich nach dem erfindungsgemäßen Verfahren (b) herstellen lassen.

Die bei der Durchführung des erfindungsgemäßen Verfahrens (c, Variante α) als Reaktionskomponenten benötigten Amino-Verbindungen sind durch die Formel (V) allgemein definiert. In dieser Formel steht R⁷ vorzugsweise für Wasserstoff oder Alkyl mit 1 bis 4 Kohlenstoffatomen, besonders bevorzugt für Wasserstoff, Methyl oder Ethyl.

Als Reaktionskomponenten in Betracht kommen auch Säureadditions-Salze, vorzugsweise Chlorwasserstoff-Additions-Salze von Amino-Verbindungen der Formel (V).

Sowohl die Amino-Verbindungen der Formel (V) als auch deren Säureadditions-Salze sind bekannt oder lassen sich nach bekannten Methoden herstellen.

Als Verdünnungsmittel kommen bei der Durchführung des erfindungsgemäßen Verfahrens (c, Variante α) alle üblichen inerten, organischen Solventien in Frage. Vorzugsweise verwendbar sind Alkohole, wie Methanol, Ethanol, n-Propanol oder Isopropanol.

Als Katalysatoren kommen bei der Durchführung des erfindungsgemäßen Verfahrens (c, Variante α) alle für derartige Umsetzungen üblichen Reaktionsbeschleuniger in Betracht. Vorzugsweise verwendbar sind saure oder basische Katalysatoren, wie z.B. der unter der Bezeichnung Amberlyst A-21^{®} im Handel befindliche, schwach basische Ionenaustaucher.

Die Reaktionstemperaturen können bei der Durchführung des erfindungsgemäßen Verfahrens (c, Variante α) innerhalb eines bestimmten Bereiches variiert werden. Im Allgemeinen arbeitet man bei Temperaturen zwischen 0°C und 80°C, vorzugsweise zwischen 10°C und 60°C.

Bei der Durchführung des erfindungsgemäßen Verfahrens (c, Variante α) setzt man auf 1 mol an Pyrazolopyrimidin der Formel (1b) im Allgemeinen eine äquivalente Menge oder einen Überschuss, vorzugsweise zwischen 1,1 und 1,5 mol an Amino-Verbindung der Formel (V) oder eines Säureadditions-Salzes davon ein. Die Aufarbeitung erfolgt nach üblichen Methoden. Im Allgemeinen geht man so vor, dass man das Reaktionsgemisch gegebenenfalls filtriert, dann einengt und reinigt.

Die bei der Durchführung des erfindungsgemäßen Verfahrens (c, Variante β) als Reaktionskomponenten benötigten Triphenylphosphonium-Salze sind durch die Formel (VI) allgemein definiert. In dieser Formel steht Ph für Phenyl. R⁸ steht vorzugsweise für Wasserstoff oder Alkyl mit 1 bis 4 Kohlenstoffatomen, wobei die Alkyl-Reste durch Carboxyl, Methoxycarbonyl oder Ethoxycarbonyl substituiert sein können. Besonders bevorzugt steht R⁸ für Wasserstoff, Methyl oder Ethyl, wobei die beiden letztgenannten Reste durch Carboxyl, Methoxycarbonyl oder Ethoxycarbonyl substituiert sein können.

Die Triphenylphosphonium-Salze der Formel (VI) sind bekannt oder lassen sich nach bekannten Methoden herstellen.

Als Basen kommen bei der Durchführung des erfindungsgemäßen Verfahrens (c, Variante β) alle für derartige Wittig-Reaktionen üblichen Deprotonierugsmittel in Betracht. Vorzugsweise verwendbar ist Butyl-lithium.

Als Verdünnungsmittel kommen bei der Durchführung des erfindungsgemäßen Verfahrens (c, Variante β) alle für derartige Wittig-Reaktionen üblichen organischen Solventien in Frage. Vorzugsweise verwendbar sind Ether, wie Dioxan oder Tetrahydrofuran.

Die Reaktionstemperaturen können bei der Durchführung des erfindungsgemäßen Verfahrens (c, Variante β) innerhalb eines bestimmten Bereiches variiert werden. Im Allgemeinen arbeitet man bei Temperaturen zwischen -78°C und +30°C.

Bei der Durchführung des erfindungsgemäßen Verfahrens (c, Variante β) setzt man auf 1 mol an Pyrazolopyrimidin der Formel (Ib) eine äquivalente Menge oder auch einen Überschuss an Triphenylphosphonium-Salz der Formel (VI) sowie eine äquivalente Menge oder auch einen Überschuss an Base ein. Die Aufarbeitung erfolgt nach üblichen Methoden.

Die bei der Durchführung des erfindungsgemäßen Verfahrens (c, Variante γ) als Reaktionskomponenten benötigten Alkylierungsmittel sind durch die Formel (VII) allgemein definiert. In dieser Formel steht R⁹ vorzugsweise für Alkyl mit 1 bis 4 Kohlenstoffatomen, besonders bevorzugt für Methyl oder Ethyl. X³ steht vorzugsweise für Chlor, Brom, Iod oder den Rest R⁹-O-SO₂-O-, worin R⁹ die zuvor angegebenen Bedeutungen hat.

Die Alkylierungsmittel der Formel (VII) sind bekannt oder lassen sich nach bekannten Methoden herstellen.

Verwendet man bei der Durchführung des erfindungsgemäßen Verfahrens (c, Variante γ) in der ersten Stufe Di-isobutyl-aluminiumhydrid als Reduktionsmittel, so arbeitet man zweckmäßigerweise unter den Bedingungen, die bereits im Zusammenhang mit der Beschreibung des erfindungsgemäßen Verfahrens (b, Variante α) erwähnt wurden.

Verwendet man bei der Durchführung des erfindungsgemäßen Verfahrens (c, Variante γ) in der ersten Stufe Natriumborhydrid als Reduktionsmittel, so verwendet man als Verdünnungsmittel im Allgemeinen Alkohole, vorzugsweise Methanol, Ethanol oder Isopropanol.

Bei der Reduktion mit Natriumborhydrid können die Reaktionstemperaturen innerhalb eines bestimmten Bereiches variiert werden. Im Allgemeinen arbeitet man bei Temperaturen zwischen 0°C und 70°C, vorzugsweise zwischen 0°C und 50°C.

Bei der Durchführung der Reduktion mit Natriumborhydrid setzt man auf 1 mol an Pyrazolopyrimidin der Formel (Ib) eine äquivalente Menge oder auch einen Überschuss an Natriumborhydrid ein. Die Aufarbeitung erfolgt wiederum nach üblichen Methoden.

Bei der Durchführung der zweiten Stufe des erfindungsgemäßen Verfahrens (c, Variante γ) kommen als Basen alle üblichen Säurebindemittel in Frage. Vorzugsweise verwendbar sind Alkalimetall-hydride, -alkoholate und -carbonate, wie Natriumhydrid, Natriummethylat, Kalium-tert.-butylat, Natriumcarbonat, Kaliumcarbonat oder Lithiumcarbonat.

Als Verdünnungsmittel kommen bei der Durchführung der zweiten Stufe des erfindungsgemäßen Verfahrens (c, Variante γ) alle üblichen, inerten organischen Solventien in Betracht. Vorzugsweise verwendbar sind Ether, wie Dioxan oder Tetrahydrofuran, und außerdem Nitrile, wie Acetonitril.

Die Temperaturen können bei der Durchführung der zweiten Stufe des erfindungsgemäßen Verfahrens (c, Variante γ) innerhalb eines größeren Bereiches variiert werden. Im Allgemeinen arbeitet man bei Temperaturen zwischen 0°C und 100°C, vorzugsweise zwischen 20°C und 80°C.

Bei der Durchführung der zweiten Stufe des erfindungsgemäßen Verfahrens (c, Variante γ) setzt man auf 1 mol an Pyrazolopyrimidin der Formel (Ic) im Allgemeinen 1 bis 2 mol, vorzugsweise 1 bis 1,5 mol an Alkylierungsmittel ein. Die Aufarbeitung erfolgt wiederum nach üblichen Methoden.

Die bei der Durchführung des erfindungsgemäßen Verfahrens (d) als Ausgangsstoffe benötigten Pyrazolopyrimidine sind durch die Formel (Id) allgemein beschrieben. In dieser Formel haben R¹, R², R³, R⁴ und Hal vorzugsweise diejenigen Bedeutungen, die bereits im Zusammenhang mit der Beschreibung der erfindungsgemäßen Stoffe der Formel (I) für diese Reste als bevorzugt genannt wurden. R¹⁰ steht vorzugsweise für Wasserstoff oder Alkyl mit 1 bis 4 Kohlenstoffatomen, besonders bevorzugt für Wasserstoff, Methyl, Ethyl oder Propyl, wobei die drei zuletzt genannten Reste jeweils substituiert sein können durch Carboxyl, Methoxycarbonyl oder Ethoxycarbonyl.

Bei den Pyrazolopyrimidinen der Formel (Id) handelt es sich um erfindungsgemäße Stoffe, die sich nach dem erfindungsgemäßen Verfahren (a) herstellen lassen.

Nach einer besonderen Verfahrensvariante lassen sich die Pyrazolopyrimidine herstellen, indem man

### k) Pyrazolopyrimidine der Formel

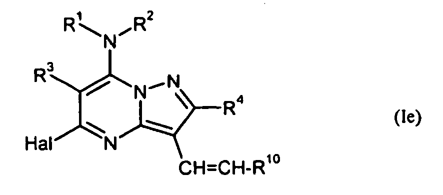

in welcher
R¹, R², R³, R⁴, R¹⁰ und Hal die oben angegebenen Bedeutungen haben,
mit Brom in Gegenwart eines inerten, organischen Verdünnungsmittels, wie Dichlormethan, Trichlormethan oder Tetrachlormethan, bei Temperaturen zwischen -20°C und +20°C umsetzt. Die Reaktionskomponenten werden dabei vorzugsweise in annähernd äquivalenten Mengen eingesetzt. Die Aufarbeitung erfolgt nach üblichen Methoden.

Als starke Basen kommen bei der Durchführung des erfindungsgemäßen Verfahrens (d) vorzugsweise Alkalilmetallalkoholate in Frage, wobei Natriummethylat und Kalium-tert.-butylat beispielhaft genannt seien.

Als Verdünnungsmittel kommen bei der Durchführung des erfindungsgemäßen Verfahrens (d) alle für derartige Umsetzungen üblichen, inerten, organischen Solventien in Betracht. Vorzugsweise verwendbar sind Alkohole, wie Methanol oder Ethanol, sowie Nitrile, wie Acetonitril.

Die Temperaturen können bei der Durchführung des erfindungsgemäßen Verfahrens (d) innerhalb eines bestimmten Bereiches variiert werden. Im Allgemeinen arbeitet man bei Temperaturen zwischen -10°C und +80°C, vorzugsweise zwischen 0°C und 60°C.

Bei der Durchführung des erfindungsgemäßen Verfahrens (d) setzt man auf 1 mol an Pyrazolopyrimidin der Formal (Id) im Allgemeinen 2 bis 3.Äquivalente oder auch einen höheren Überschuss am starker Base ein. Die Aufarbeitung erfolgt wiederum nach üblichen Methoden.

Die bei der Durchführung des Verfahrens (e) als Ausgangsstoffe benötigten Pyrazolopyrimidine sind durch die Formel (VIII) allgemein definiert. In dieser Formel haben R¹, R², R³, R⁴ und Hal vorzugsweise diejenigen Bedeutungen, die bereits im Zusammenhang mit der Beschreibung der erfindungsgemäßen Stoffe der Formel (I) für diese Reste als bevorzugt angegeben wurden.

Die Pyrazolopyrimidine der Formel (VIII) sind bekannt oder lassen sich nach bekannten Methoden herstellen (vgl. PCT/EP 03/05 159).

Die bei der Durchführung des Verfahrens (e) als Reaktionskomponenten benötigten Acyl-Derivaten sind durch die Formel (IX) allgemein definiert. In dieser Formel steht R¹¹ vorzugsweise für Alkyl mit 1 bis 4 Kohlenstoffatomen, besonders bevorzugt für Methyl, Ethyl oder n-Propyl. X⁴ steht vorzugsweise für Chlor oder einen Rest der Formel 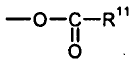 worin R¹¹ wiederum die zuvor angegebene Bedeutung hat.

Die Acyl-Derivate der Formel (IX) sind bekannt oder lassen sich nach bekannten Methoden herstellen.

Als Katalysatoren kommen bei der Durchführung des erfindungsgemäßen Verfahrens (e) alle derartige Friedel-Crafts-Reaktionen üblichen Reaktionsbeschleuniger in Betracht. Vorzugsweise verwendbar sind Metallchloride, wie Aluminiumtrichlorid oder Eisen(III)chlorid.

Als Verdünnungsmittel kommen bei der Durchführung des erfindungsgemäßen Verfahrens (e) alle für derartige Umsetzungen üblichen, inerten organischen Solventien in Frage. Vorzugsweise verwendbar sind Ether, wie Diethylether, Dioxan oder Tetrahydrofuran.

Die Temperaturen können bei der Durchführung des erfindungsgenäßen Verfahrens (e) innerhalb eines bestimmten Bereiches variiert werden. Im Allgemeinen arbeitet man bei Temperaturen zwischen -20°C und +20°C, vorzugsweise zwischen -10°C und +10°C.

Bei der Durchführung des erfindungsgemäßen Verfahrens (e) setzt man auf 1 mol an Pyrazolopyrimidin der Formel (VIII) im Allgemeinen 2 bis 5 mol an Acyl-Derivat der Formel (IX) sowie eine entsprechende Menge an Katalysator ein. Die Aufarbeitung erfolgt nach üblichen Methoden.

Als Verdünnungsmittel kommen bei der Durchführung des erfindungsgemäßen Verfahrens (f) alle für derartige Halogenierungen üblichen Solventien in Frage. Vorzugsweise verwendbar sind halogenierte aliphatische oder aromatische Kohlenwasserstoffe, wie Chlorbenzol. Als Verdünnungsmittel kann aber auch das Halogenierungsmittel selbst, z.B. Phosphoroxychlorid oder ein Gemisch von Halogenierungsmitteln fungieren.

Die Temperaturen können auch bei der Durchführung des Verfahrens (f) in einem größeren Bereich variiert werden. Im Allgemeinen arbeitet man bei Temperaturen zwischen 0°C und 150°C, vorzugsweise zwischen 10°C und 120°C.

Bei der Durchführung des Verfahrens (f) setzt man Hydroxy-pyrazolo-pyrimidin der Formel (X) im Allgemeinen mit einem Überschuss an Halogenierungsmittel um. Die Aufarbeitung erfolgt nach üblichen Methoden.

Als Verdünnungsmittel kommen bei der Durchführung des Verfahrens (h) alle für derartige Umsetzungen üblichen, inerten organischen Solventien in Frage. Vorzugsweise verwendbar sind Alkohole, wie Methanol, Ethanol, n-Propanol, i-Propanol, n-Butanol und tert.-Butanol.

Als Säurebindemittel kommen bei der Durchführung des Verfahrens (h) alle für derartige Umsetzungen üblichen anorganischen und organischen Basen in Betracht Vorzugsweise verwendbar sind tertiäre Amine, wie Tributylamin oder Pyridin. Im Überschuss eingesetztes Amin kann auch als Verdünnungsmittel fungieren.

Die Temperaturen können bei der Durchführung des Verfahrens (h) in einem größeren Bereich variiert werden. Im Allgemeinen arbeitet man bei Temperaturen zwischen 20°C und 200°C, vorzugsweise zwischen 50°C und 180°C.

Bei der Durchführung des Verfahrens (h) setzt man Heterocyclylmalonester der Formel (XII) und

Aminopyrazol der Formel (XIII) im Allgemeinen in äquivalenten Mengen um. Es ist aber auch möglich, die eine oder andere Komponente in einem Überschuss zu verwenden. Die Aufarbeitung erfolgt nach üblichen Methoden.

Als Verdünnungsmittel kommen bei der Durchführung der erfindungsgemäßen Verfahren (i) und (j) jeweils alle üblichen, inerten organischen Solventien in Frage. Vorzugsweise verwendbar sind halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder i-Propanol, n-, i-, sek-oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder auch reines Wasser.

Als Kupfersalze kommen bei der Durchführung der erfindungsgemäßen Verfahren (i) und (j) jeweils übliche Kupfersalze in Betracht. Vorzugsweise verwendbar sind Kupfer(I)chlorid oder Kupfer(I)bromid.

Als Säureakzeptoren kommen bei der Durchführung der erfindungsgemäßen Verfahren (i) und (j) jeweils alle üblichen anorganischen oder organischen Basen in Frage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithium-diisopropylamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat und außerdem Ammonium Verbindungen wie Ammoniumhydroxid, Ammoniumacetat und Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können auch bei der Durchführung der erfindungsgemäßen Verfahren (i) und (j) in einem größeren Bereich variiert werden. Im Allgemeinen arbeitet man bei Temperaturen zwischen 0°C und 150°C, vorzugsweise bei Temperaturen zwischen 0°C und 80°C.

Bei der Durchführung des erfindungsgemäßen Verfahrens (i) setzt man auf 1 mol an Halogenpyridin der Formel (XIV) im Allgemeinen 1 bis 15 mol, vorzugsweise 1,3 bis 8 mol an Malonester der Formel (XV) ein. Die Aufarbeitung erfolgt nach üblichen Methoden.

Bei der Durchführung des erfindungsgemäßen Verfahrens (j) setzt man auf 1 mol an Halogenpyrimidin der Formel (XVI) im Allgemeinen 1 bis 15 mol, vorzugsweise 1,3 bis 8 mol an Malonester der Formel (XV) ein. Die Aufarbeitung erfolgt wiederum nach üblichen Methoden.

Die erfindungsgemäßen Verfahren werden im Allgemeinen unter Atmosphärendruck durchgeführt. Es ist jedoch auch möglich, unter erhöhtem Druck zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von I bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten; wie beispielsweise gegen Erysiphe-Arten, von Krankheiten im Wein-, Obst- und Gemüseanbau, wie beispielsweise gegen Botrytis-, Venturia-, Sphaerotheca- und Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxychinolinsulfat;
Acibenzolar-S-methyl; Aldimorph; Amidoflumet; Ampropylfos; Ampropylfos-potassium; Andoprim; Anilazine; Azaconazole; Azoxystrobin;
Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin;
Calcium-polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram;
Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon;
Edifenphos; Epoxiconazole; Ethaboxam; Ethirimol; Etridiazole;
Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al, Fosetyl-sodium; Fuberidazole; Furalaxyl; Furametpyr; Furcarbanil; Furmecyclox;
Guazatine;
Hexachlorobenzene; Hexaconazole; Hymexazol;
Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione;
Kasugamycin; Kresoxim-methyl;
Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin;
Natamycin; Nicobifen; Nitrothal-isopropyl; Noviflumuron; Nuarimol;
Ofurace; Orysastrobin; Oxadixyl; Oxolinic acid; Oxpoconazole; Oxycarboxin; Oxyfenthiin;
Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine;
Quinconazole; Quinoxyfen; Quintozene;
Simeconazole; Spiroxamine; Sulfur;
Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole;
Uniconazole;
Validamycin A; Vinclozolin;
Zineb; Ziram; Zoxamide;
(2S)-N-[2-[4-[[3-(4-Chlorphenyl)-2-propinyl]oxy]-3-methoxyphenyl]ethyl]-3-methyl-2-[(methylsulfonyl)amino]-butanamid;
1-(1-Naphthalenyl)-1H-pyrrol-2,5-dion;
2,3,5,6-Tetrachlor-4-(methylsulfonyl)-pyridin;
2-Amino-4-methyl-N-phenyl-5-thiazolcarboxamid;
2-Chlor-N-(2,3-dihydro-1,1,3-trimethyl-1H-inden-4-yl)-3-pyridincarboxamide;
3,4,5-Trichlor-2,6-pyridincarbonitril;
Actinovate;
cis-1-(4-Chlorphenyl)-2-(1H-1,2,4-triazol-1-yl)-cycloheptanol;
Methyl 1-(2,3-dihydro-2,2-dimethyl-1H-inden-1-yl)-1H-imidazol-5-carboxylat;
Monokaliumcarbonat;
N-(6-Methoxy-3-pyridinyl)-cyclopropancarboxamid;
Natriumtetrathiocarbonat;
sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin IR-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, lodofenphos, Iprobenfos, Isazofos, Isofenphos, Isoprocarb, Isoxathion, Ivermectin,
Japonilure,
Kadethrin, Kernpolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemetonmethyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IR-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Weiterhin eignen sich die erfindungsgemäßen Verbindungen der Formel (I) zur Unterdrückung des Wachstums von Tumorzellen in Menschen und Säugetieren. Dies basiert auf einer Wechselwirkung der erfindungsgemäßen Verbindungen mit Tubulin und Mikrotubuli und durch Förderung der Mikrotubuli-Polymerisation.

Zu diesem Zweck kann man eine wirksame Menge an einer oder mehreren Verbindungen der Formel (1) oder pharmazeutisch verträglicher Salze davon verabreichen.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften (;,Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften (_{"}Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Namatoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften (_{"}Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzetibehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

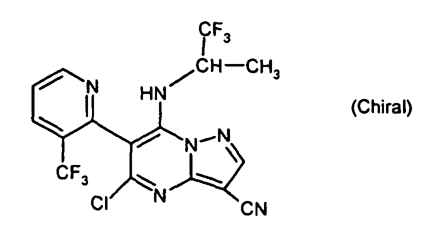

### (Verfahren a)

Zu einer Lösung von 0,2 g (0,56 mmol) 3-Cyano-5,7-dichlor-6-(3-trifluormethyl-pyridin-2-yl)-pyrazolo[1,5-a]pyrimidin in 10 ml Acetonitril gibt man 0,065 g (1,12 mmol) Kaliumfluorid, rührt 2 Stunden bei 80°C und kühlt anschließend auf 0°C ab. Zu dieser Lösung gibt man 0,13 g (1,17 mmol) (S)-2,2,2-Trifluor-isopropylamin und rührt 18 Stunden bei 80°C. Danach wird das Reaktionsgemisch auf Raumtemperatur abgekühlt und in 30 ml verdünnte Salzsäure eingerührt. Man extrahiert mit Dichlormethan, wäscht die organische Phase zweimal mit Wasser, trocknet über Natriumsulfat und engt unter vermindertem Druck ein. Der verbleibende Rückstand wird mit einer Mischung aus Petrolether/Methyl-tert.-butylether = 15:1 über eine kurze Kieselgelsäule filtriert. Man erhält auf diese Weise 0,15 g (58,5 % der Theorie) an 3-Cyano-5-chlor-6-(3-trifluormethyl-pyridin-2-yl)-pyrazolo[1,5-a]-pyrimidin-N-[(1,S)-2,2,2-trifluor-1-methylethyl]-amin.
HPLC: logP = 3,14

Nach den zuvor angegebenen Methoden werden auch die in der folgenden Tabelle 1 aufgeführten Pyrazolopyrimidine der Formel 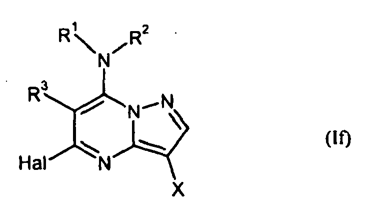 hergestellt.

**Tabelle 1**

| **Bsp.- Nr.** | **R¹** | **R²** | **R³** | **Hal** | **X** | **logP** |
|---|---|---|---|---|---|---|
| 2 | 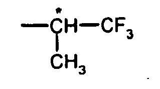 | H | 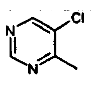 | Cl | -CN | 2,77 Isomer |
| 3 | 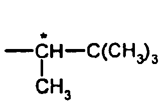 | H | 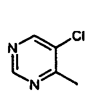 | Cl | -CN | 3,54 |
| 4 | 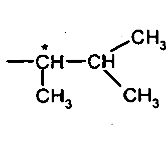 | H | 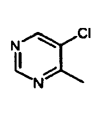 | Cl | -CN | 3,13 |
| 5 | 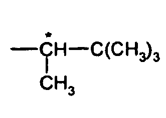 | H | 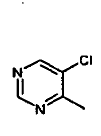 | Cl | -Cl | 4,36 |
| 6 | 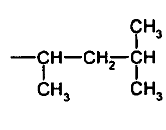 | H | 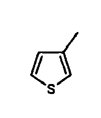 | Cl | -CN | 4,46 |
| 7 | 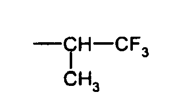 | H | 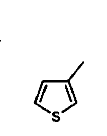 | Cl | -CN | 3,53 |
| 8 | 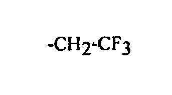 | H | 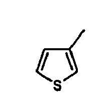 | Cl | -CN | 3,21 |
| 9 | 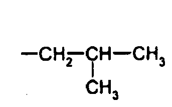 | H | 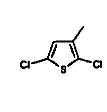 | Cl | -CN | 4,82 |
| 10 | 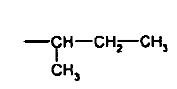 | H | 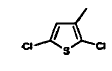 | Cl | -CN | 4,82 |
| 11 | 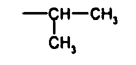 | H | 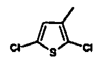 | Cl | -CN | 4,41 |
| 12 | 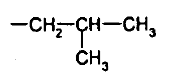 | -CH₃ | 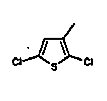 | Cl | -CN | 5,31 |
| 13 | 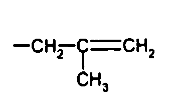 | -CH₃ | 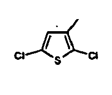 | Cl | -CN | 5,10 |
| 14 | -NH-CH₂-CH₂-CH₂-CH₂- | | 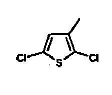 | Cl | -CN | 4,46 |
| 15 | -CH₂-C(CH₃)₃ | H | 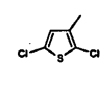 | Cl | -CN | 5,31 |
| 16 | 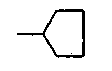 | H | 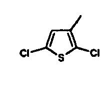 | Cl | -CN | 5,03 |
| 17 | -CH₂-CH₂-OCH₃ | H | 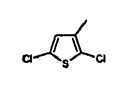 | Cl | -CN | 3,90 |
| 18 | -CH₂-CH₂-OCH₃ | -CH₃ | 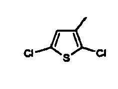 | Cl | -CN | 4,32 |
| 19 | -CH₂-CH₂-CN | -CH₃. | 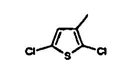 | Cl | -CN | 3,90 |
| 20 | 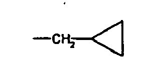 | -C₃H₇-n | 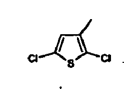 | Cl | -CN | 5,78 |
| 21 | -CH₂-CH₂-CH₂-CH₂- | | 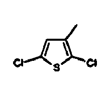 | Cl | -CN | 4,61 |
| 22 | 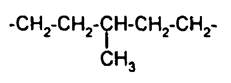 | | 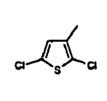 | Cl | -CN | 5,59 |
| 23 | -CH₂-CH₂-CH₂-CH₂-CH₂- | | 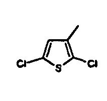 | Cl | -CN | 6,19 |
| 24 | 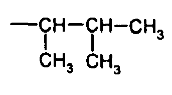 | H | 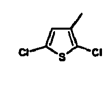 | Cl | -CN | 5,19 |
| 25 | 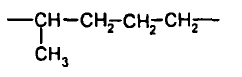 | | 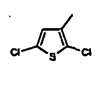 | Cl | -CN | 4,98 |
| 26 | 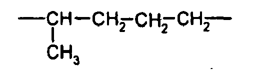 | | 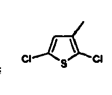 | Cl | -CN | 4,58 |
| 27 | 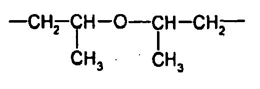 | | 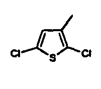 | Cl | -CN | 4,72 |
| 28 | -CH₂-CH₂-O-CH₂-CH₂- | | 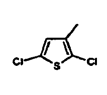 | Cl | -CN | 3,85 |
| 29 | 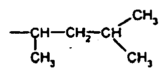 | H | 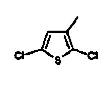 | Cl | -CN | 5,59 |
| 30 | -CH₂-CF₃ | H | 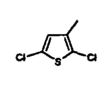 | Cl | -CN | 4,06 |
| 31 | 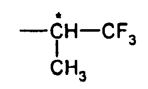 | H | 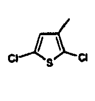 | Cl | -CN | 4,46 |
| 32 | -CH₂-C(CH₃)3 | H | 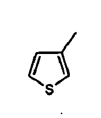 | Cl | -CN | 4,18 |
| 33 | 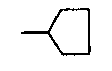 | H | 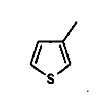 | Cl | -CN | 3,00 |
| 34 | 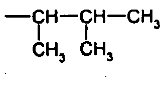 | H | 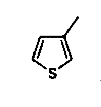 | Cl | -CN | 4,08 |
| 35 | 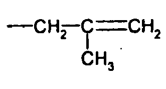 | -CH₃ | 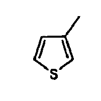 | Cl | -CN | 3,98 |
| 36 | 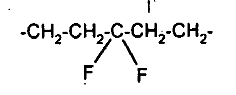 | | 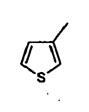 | Cl | -CN | 3,61 |
| 37 | 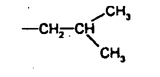 | H | 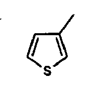 | Cl | -CN | 3,78 |
| 38 | 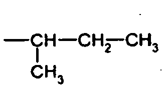 | H | 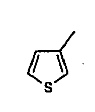 | Cl | -CN | 3,74 |
| 39 | 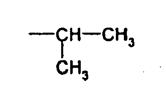 | H | 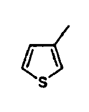 | Cl | -CN | 3,37 |
| 40 | 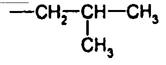 | -CH₃ | 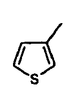 | Cl | -CN | 4,13 |
| 41 | -CH₂-CH₂-CH₂-CH₂- | | 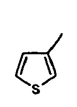 | Cl | -CN | 3,53 |
| 42 | 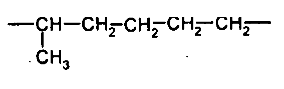 | | 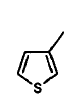 | Cl | -CN | 4,27 |
| 43 | 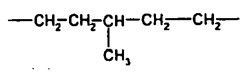 | | 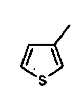 | Cl | -CN | 4,41 |
| 44 | -CH₂-CH₂-O-CH₂-CH₂- | | 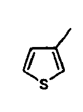 | Cl | -CN | 2,90 |
| 45 | -CH₂-CH₂-OCH₃ | H | 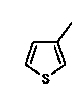 | Cl | -CN | 2,94 |
| 46 | 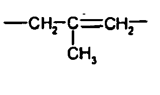 | -CH₂-CH₃ | 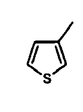 | Cl | -CN | 4,32 |
| 47 | 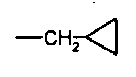 | -CH₂-CH₂-CH₃ | 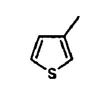 | Cl | -CN | 4,51 |
| 48 | 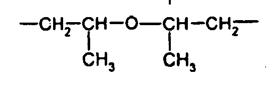 | | 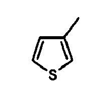 | Cl | -CN | 3,53 |
| 49 | -CH₂-CH₂-CH₂-CH₂-CH₂- | | 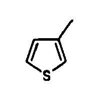 | Cl | -CN | 3,58 |
| 50 | -N-CH₂-CH₂-CH₂-CH₂-H | | 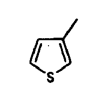 | Cl | -CN | 3,15 |
| 51 | -CH2-CH₂-OCH₃ | -CH₃ | 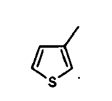 | Cl | -CN | 3,17 |
| 52 | -CH₂-CH₂-CN | -CH₂-CH₃ | 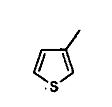 | Cl | -CN | 2,98 |
| 53 | 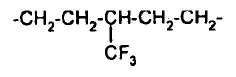 | | 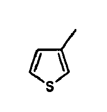 | Cl | -CN | 4,03 |
| 54 | -CH(CH₃)-CH₂-CH₂-CH₂- | | 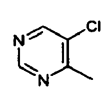 | Cl | -CHO | 2,44 |
| 55 | 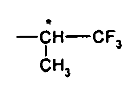 | H | 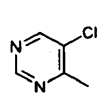 | Cl | -CHO | ,2,46 |
| 56 | 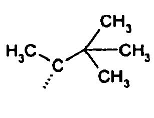 | H | 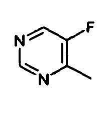 | Cl | -CN | 3,29 |
| 57 | 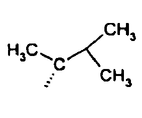 | H | 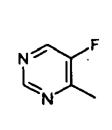 | Cl | -CN | 2,92 |
| 58 | 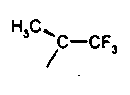 | H | 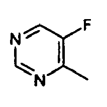 | Cl | -CN | 2,65 |
| 59 | 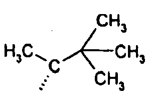 | H | 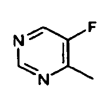 | Cl | Cl | 4,11 |
| 60 | 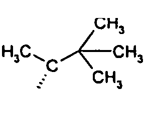 | H | 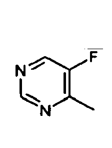 | Cl | H | 3,42 |
| 61 | 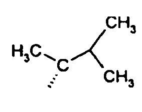 | H | 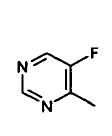 | Cl | H | 2,98 |
| 62 | 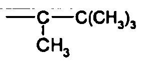 | H | 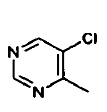 | Cl | -CHO | 3,12 |
| 63 | -CH₂-CH₂-CH(CH₃)-CH₂-CH₂ | | 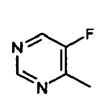 | Cl | -CN | 3,20 |
| 64 | 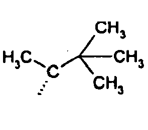 | H | 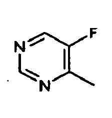 | Cl | -COOCH₃ | 3,15 |

### Herstellung von Vorprodukten der Formel (II):

### Beispiel 65

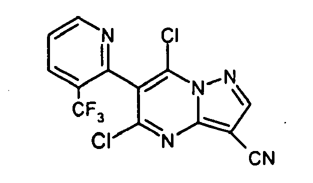

### Verfahren (f)

In ein Gemisch aus 5,8 g (18,1 mmol) 3-Cyano-6-(3-trifluormethyl-pyridin-2-yl)-pyrazolo[1,5-a]pyrimidin-5,7-diol und 22,15 g (144,5 mmol) Phosphoroxychlorid werden bei Raumtemperatur unter Rühren 3,0 g (14,5 mmol) Phosphorpentachlorid in 5 Portionen gegeben. Das Reaktionsgemisch wird 4 Stunden unter Rückfluss erhitzt, dann auf Raumtemperatur abgekühlt und unter vermindertem Druck eingeengt. Der verbleibende Rückstand wird mit 100 ml Wasser versetzt und danach dreimal mit je 100 ml Dichlormethan extrahiert. Die vereinigten organischen Phasen werden zweimal mit je 50 ml Wasser gewaschen, über Natriumsulfat getrocknet und unter vermindertem Druck eingeengt. Der verbleibende Rückstand wird mit Hexan/Essigsäureethylester = 3:1 an Kieselgel chromatografiert. Man 0,88 g (14,8 % der Theorie) an 3-Cyano-5,7-dichlor-6-(3-trifluormethyl-pyridin-2-yl)-pyrazolo[1,5-a]pyrimidin.
HPLC: logP = 2,68

### Beispiel 66

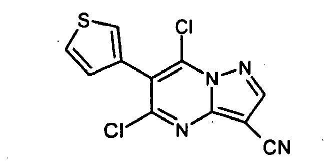

Ein Gemisch aus 2,0g (10,74 mmol) 2-Thienyl-malonsäure und 1,16 g (10,74 mmol) 3-Amino-4-cyano-pyrazol wird bei Raumtemperatur unter Rühren innerhalb von 2 Minuten mit 41,13 g (268 mmol) Phosphoroxychlorid versetzt. Danach wird 18 Stunden auf 90°C erhitzt und dann auf Raumtemperatur abgekühlt. Das Reaktionsgemisch wird in 250 ml Eiswasser gegeben, und die dabei entstehende Suspension wird 1 Stunde gerührt. Man saugt ab und wäscht mit 50 ml Wasser. Zur weiteren Reinigung wird das Produkt in 50 ml Cyclohexan/Essigsäureethylester = 1:1 suspendiert und kurz aufgekocht, dann abgekühlt, über eine kurze Kieselsäule abgesaugt und 8 mal mit je 50 ml Cyclohexan/Essigsäureethylester = 1:1 gewaschen. Das Filtrat wird über Natriumsulfat getrocknet und dann erneut filtriert. Der Filter-Rückstand wird mit wenig Cyclohexan/Essigsäureethylester = 1:1 nachgewaschen. Das gesamte Filtrat wird unter vermindertem Druck eingeengt. Man erhält 1,48 g (30,34 % der Theorie) an 5,7-Dichlor-3-cyano-6-(thien-3-yl)-pyrazolo-[1,5-a]pyrimidin in Form eines Feststoffes.

### Beispiel 67

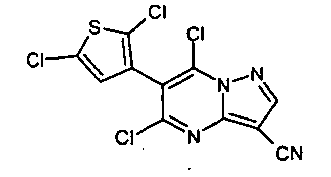

In eine Lösung von 7,5 g (25,41 mmol) 5,7-Dichlor-3-cyano-6-(thien-3-yl)-pyrazolo[1,5-a]pyrimidin in 80 ml Dichlormethan wird bei Temperaturen zwischen -5°C und 0°C zwei Stunden lang ein Chlorgasstrom eingeleitet. Danach wird das Reaktionsgemisch auf Raumtemperatur erwärmt und dann unter verminderten Druck eingeengt. Man nimmt den verbleibenden Rückstand mit Dichlormethan auf und saugt ab. Dabei erhält man 2,0 g an dem gewünschten Produkt. Das zuvor aufgefangene Filtrat wird nach dem Einengen mit Cyclohexan/Essigsäureethylester = 1:1 an Kieselgel chromatographiert. Nach dem Einengen des Eluates werden erneut 3,5 g des gewünschten Produktes isoliert. Man erhält auf diese Weise insgesamt 5,5 g (54,13 % der Theorie) an 5,7-Dichlor-3-cyano-(2,5-dichlor-thien-3-yl)-pyrazolo[1,5-a]pyrimidin

### Herstellung von Vorprodukt der Formel (X):

### Beispiel 68

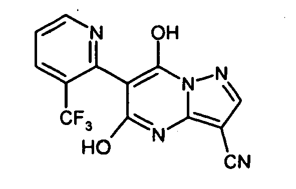

### Verfahren (h)

Ein Gemisch aus 4,1 g (14,8 mmol) 2-(3-Trifluormethyl-pyridin-2-yl)-malonsäuredimethylester, 1,6 g (14,8 mmol) 3-Amino-4-cyano-pyrazol und 3,02 g (16,3 mmol) Tri-n-butylamin wird 2 Stunden unter Rühren auf 180°C erhitzt. Dabei wird das während der Umsetzung entstehende Methanol kontinuierlich abdestilliert. Anschließend wird das Reaktionsgemisch auf Raumtemperatur abgekühlt. Das sich abscheidende Tri-n-butylamin wird abdekantiert, und das verbleibende Gemisch wird unter vermindertem Druck andestilliert. Man erhält 5,8 g eines Produktes, das gemäß HPLC zu 60 % aus 3-Cyano-6-(3-trifluormethyl-pyridin-2-yl)-pyrazolo[1,5-a]pyrimidin-5,7-diol besteht. Die Ausbeute errechnet sich danach zu 73,25 % der Theorie. Das Produkt wird ohne zusätzliche Reinigung für weitere Synthesen verwendet.
HPLC: logP = 0,29

### Herstellung von Vorprodukt der Formel (XII-a):

### Beispiel 69

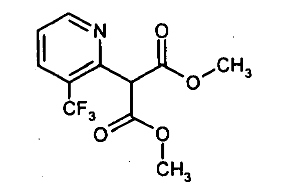

### Verfahren (i)

9 g (207 mmol) 60%ige Natriumhydridsuspension werden in 300 ml Dioxan suspendiert. Hierzu tropft man bei 55-60°C 27,29 g (206,6 mmol) Malonsäuredimethylester und rührt weitere 30 Minuten bei gleicher Temperatur. Nach Zugabe von 8,18 g (82,63 mmol) Kupfer(I)chlorid erwärmt man auf 80°C und tropft dann 15 g (82,63 mmol) 2-Chlor-3-trifluormethylpyridin hinzu. Die Reaktionsmischung wird nun noch 14 Stunden bei 100°C gerührt. Nach dem anschließenden Abkühlen auf 15-20°C tropft man langsam konzentrierte Salzsäure zu bis die Mischung sauer reagiert. Nun gibt man 600 ml Wasser und 300 ml Dichlormethan hinzu und filtriert unlösliche Bestandteile ab. Von dem Filtrat wird die organische Phase abgetrennt, über Natriumsulfat getrocknet und unter vermindertem Druck eingeengt. Der Rückstand wird mit Hexan/Essigester (4:1) an Kieselgel chromatografiert. Man erhält 10,1 g (40% der Theorie) an 2-[(3-Trifluormethyl)-pyrimidin-2-yl]-malonsäuredimethylester.
HPLC: logP = 2,05

### Herstellung von Vorprodukt der Formel (XII-b):

### Beispiel 70

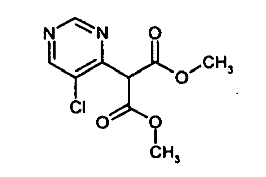

### Verfahren (j)

2,6 g (65,4 mmol) 60%ige Natriumhydridsuspension werden in 100 ml Tetrahydrofuran suspendiert. Hierzu gibt man bei 0°C 6,9 g (52,4 mmol) Malonsäuredimethylester und rührt 0,5 Stunden bei gleicher Temperatur. Anschließend tropft man eine Lösung von 6,5 g (43,63 mmol) 4,5-Dichlorpyrimidin in 50 ml Tetrahydrofuran hinzu und rührt weitere 3 Stunden bei Raumtemperatur. Danach tropft man langsam 150 ml 1N Salzsäure zu und extrahiert danach mit 100 ml Dichlormethan. Die organische Phase wird abgetrennt, über Natriumsulfat getrocknet und unter vermindertem Druck eingeengt. Der Rückstand wird mit Methyl-t-butylether/Petrolether (1:9) an Kieselgel chromatografiert. Man erhält 7 g (65,6 % der Theorie) an 2-(5-Chlor-pyrimidin-4-yl)-malonsäuredirnethylester.
HPLC: logP= 1,33

### Beispiel 71

### Herstellung von 4,5-Dichlorpyrimidin

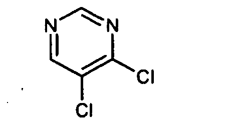

Zu einer Lösung von 112,5 g (673,7 mmol) 5-Chlor-6-oxo-1,6-dihydropyrimidin-1-ium chlorid in 630 ml Phosphoroxychlorid gibt man 1,6 ml Dimethylamin und erhitzt 3 Stunden unter Rückfluss. Danach wird das überschüssige Phosphoroxychlorid unter vermindertem Druck abdestilliert. Nach dem Abkühlen gießt man den Rückstand auf 1,51 Eiswasser, extrahiert mit 500 ml Dichlormethan, trocknet die organische Phase über Natriumsulfat und engt unter vermindertem Druck ein. Man erhält 72,3 g (66,3 % der Theorie) an 4,5-Dichlorpyrimidin.
HPLC: logP = 1,35

### Beispiel 72

### Herstellung von 5-Chlor-6-oxo-1,6-dihydropyrimidin-1-ium chlorid

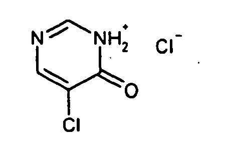

Zu einer Lösung von 77 g (0,8 mol) 4(3H)-Pyrimidinon in 770 ml Eisessig gibt man 6,5 g (40 mmol) Eisen-III-chlorid und leitet innerhalb von 2 Stunden bei 40-45°C 113,6 g (1,6 mol) Chlor ein. Die Reaktionsmischung wird auf 15°C abgekühlt, das entstandene Festprodukt abgesaugt und mit Ether gewaschen. Man erhält 112,5 g (84 % der Theorie) an 5-Chlor-6-oxo-1,6-dihydro-pyrimidin-1-ium chlorid.

### Beispiel 73

### Herstellung von 4(3H)-Pvrimidinon

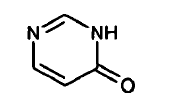

Eine Mischung von 103 g (0,804 mol) 6-Mercapto-4(1H)-pyrimidinon (JP 50053381, Chem. Abstr. CAN 84:17404) und 141,5 g (1,2 mol) Raney Nickel in 1,2 I Ethanol wird 8 Stunden unter Rückfluss erhitzt. Die Lösung wird heiß filtriert, der Rückstand mit Ethanol gewaschen und das Filtrat unter vermindertem Druck eingeengt. Man erhält 67,2 g (87 % der Theorie) an 4(3H)-Pyrimidinon.
*) Die Bestimmung der logP-Werte erfolgte gemäß EEC-Directive 79/831 Annex V. A8 durch HPLC (Gradientenmethode, Acetonitril/0,1 % wässrige Phosphorsäure)

### Beispiel 74

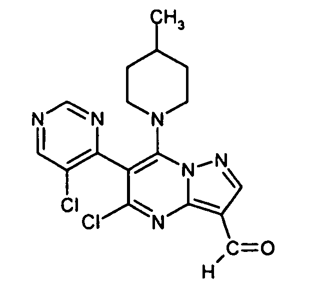

Ein Gemisch aus 5 mmol 5,7-Dichlor-6-(5-chlor-pyrimidin-4-yl)-3-formyl-pyrazolo[1,5-a]-pyrimidin, 5 mmol 4-Methylpiperidin und 5 mmol Kaliumcarbonat in 30 ml Acetonitril wird 15 Stunden bei Raumtemperatur gerührt. Danach wird das Reaktionsgemisch in 120 ml Wasser gegeben. Man extrahiert dreimal mit Essigsäureethylester, trocknet die vereinigten organischen Phasen über Natriumsulfat und engt unter vermindertem Druck ein. Der verbleibende Rückstand wird mit Cyclohexan:Essigsäureethylester = 3:1 an Kieselgel chromatographiert. Man erhält auf diese Weise 1,15 mmol an 5-Chlor-6-(5-chlor-pyrimidin-4-yl)-3-formyl-7-(4-methyl-piperidin-1-yl)-pyrazolo[1,5-a]-pyrimidin.
HPLC: log P = 3,04

### Beispiel 75

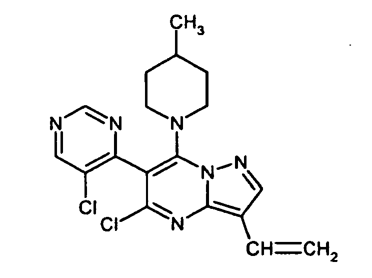

In eine Lösung aus 1,4 mmol Methyltriphenyl-phosphonium-bromid und 1,4 mmol n-Butyl-lithium in 58 ml Tetrahydrofuran werden bei -70°C unter Rühren 1,3 mmol 5-Chlor-6-(5-chlor-pyrimidin-4-yl)-3-formyl-7-(4-methyl-piperidin-1-yl)-pyrazolo[1,5-a]pyrimidin gegeben. Man rührt 15 Stunden bei Raumtemperatur nach, destilliert dann das Lösungsmittel unter vermindertem Druck ab und versetzt den Rückstand mit Wasser.

Das entstehende Gemisch wird dreimal mit Essigsäureethylester extrahiert. Die vereinigten organischen Phasen werden über Natriumsulfat getrocknet und dann unter vermindertem Druck eingeengt. Der verbleibende Rückstand wird mit Cyclohexan : Essigsäureethylester = 7:3 an Kieselgel chromatograhiert. Man erhält auf diese Weise 0,2 mmol an 5-Chlor-6-(5-chlor-pyrimidin-4-yl)-3-ethenyl-7-(4-methyl-piperidin-4-yl)-pyrazolo[1,5-a]pyrimidin.
HPLC: log P = 4,70

### Beispiel 76

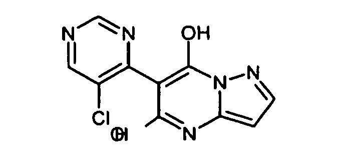

100 mmol 3-Amino-pyrazol und 100 mmol 2-(5-Chlor-pyrimidin-4-yl)-malonsäure-dimethylester werden unter Rühren bei Raumtemperatur in 27 ml Tri-n-butyl-amin gegeben. Nach beendeter Zugabe wird das Reaktionsgemisch 3 Stunden unter Rühren auf 185°C erhitzt. Dabei wird das während der Umsetzung gebildete Methanol kontinuierlich abdestilliert. Danach lässt man auf Raumtemperatur abkühlen, dekantiert von Tri-n-butyl-amin ab, verrührt den Rückstand mit einem Gemisch aus Isopropanol und Methyl-tert.-butyl-ether und dekantiert anschließend erneut. Unter vermindertem Druck werden noch enthaltene Reste an Lösungsmittel entfernt. Das erhaltene 5,7-Dihydroxy-6-(5-chlor-pyrimidin-4-yl)-pyrazolo[1,5-a]pyrimidin wird ohne zusätzliche Reinigung für die weitere Umsetzung verwendet.

### Beispiel 77

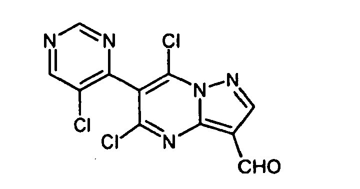

Ein Gemisch aus 56 mmol 5,7-Dihydroxy-6-(5-chlor-pyrimidin-4-yl)-pyrazolo[1,5-a]pyrimidin und 560 mmol Phosphoroxychlorid wird 30 Minuten bei 30°C gerührt, dann auf 0°C abgekühlt und dann unter Rühren tropfenweise mit 85 mmol Dimethylformamid versetzt. Nach beendeter Zugabe wird das Reaktionsgemisch zunächst 12 Stunden bei Raumtemperatur gerührt und dann 6 Stunden unter Rückfluss erhitzt. Anschließend wird das Reaktionsgemisch mit 56 mmol Phosphorpentachlorid versetzt und weitere 12 Stunden unter Rückfluss erhitzt. Nach dem Abkühlen auf Raumtemperatur wird das Reaktionsgemisch unter vermindertem Druck eingeengt und dann auf Eiswasser gegeben. Das entstehende Gemisch wird dreimal mit Essigsäurethylester extrahiert. Die vereinigten organischen Phasen werden über Natriumsulfat getrocknet und dann unter vermindertem Druck eingeengt. Das erhaltene 3-Formyl-5,7-dichlor-6-(5-chlor-pyrimidin-4yl)-pyrazolo-[1,5-a]pyrimidin wird ohne zusätzliche Reinigung für die weitere Synthese verwendet.

### Beispiel A

Venturia - Test (Apfel) / protektiv
- Lösungsmittel :: 24,5 Gewichtsteile Aceton
24,5 Gewichtsteile Dimethylacetamid
- Emulgator :: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100% relativer Luftfeuchtigkeit in einer Inkubationskabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigen die in den Beispielen 1, 2, 3, 4 und 5 aufgeführten erfindungsgemäßen Stoffe bei einer Aufwandmenge von 100 g/ha einen Wirkungsgrad von über 90 %.

### Beispiel B

Botrytis - Test (Bohne) / protektiv
- Lösungsmittel :: 24,5 Gewichtsteile Aceton
24,5 Gewichtsteile Dimethylacetamid
- Emulgator :: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigen die in den Beispielen 2, 3 und 5 aufgeführten erfindungsgemäßen Stoffe bei einer Aufwandmenge von 500 g/ha einen Wirkungsgrad von über 85 %.

### Beispiel C

Puccinia- Test (Weizen) / protektiv
- Lösungsmittel :: 50 Gewichtsteile N,N-Dimethylacetamid
- Emulgator :: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von *Puccinia recondita* besprüht. Die Pflanzen verbleiben 48Stunden bei 20°C und 100% relativer Luftfeuchtigkeit in einer Inkubationskabine.

Die Pflanzen werden dann im Gewächshaus bei einer Temperatur von ca. 20°C und einer relativen Luftfeuchtigkeit von 80% aufgestellt, um die Entwicklung von Rostpusteln zu begünstigen.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigen die in den Beispielen 2 und 39 aufgeführten erfindungsgemäßen Stoffe bei einer Aufwandmenge von 500 g/ha einen Wirkungsgrad von über 85 %.

### Beispiel D

Podosphaera - Test (Apfel) / protektiv
- Lösungsmittel :: 24,5 Gewichtsteile Aceton
24,5 Gewichtsteile Dimethylacetamid
- Emulgator :: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers *Podosphaera leucotricha* inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigen die in den Beispielen 3 und 5 aufgeführten erfindungsgemäßen Stoffe bei einer Aufwandmenge von 100 g/ha einen Wirkungsgrad von über 90 %.

### Beispiel E

### In vitro-Test zur Bestimmung der ED₅₀ an Mikroorganismen

- Lösungsmittel:: Methanol
- Emulgator:: Alkylaryl-polyglykolether

Man vermischt 2 mg Wirkstoff mit 100 µl Methanol und verdünnt das so hergestellte Konzentrat dann mit einem Gemisch aus 1000 ml Methanol und 6 g des oben angegebenen Emulgators auf die jeweils gewünschte Konzentration.

Jeweils 10 µl der Zubereitung werden in die Kavitäten von Mikrotiterplatten einpipettiert. Nachdem das Lösungsmittel verdampft ist, werden je Kavität jeweils 200 µl eines Potato-Dextrose Mediums hinzugefügt, das zuvor mit der jeweils gewünschten Konzentration an Sporen bzw. Myzel des zu prüfenden Mikroorganismus versetzt worden war. Die resultierenden Konzentrationen an Wirkstoff in den Kavitäten betragen
0,1 ppm
1 ppm
10 ppm
bzw. 100 ppm.

Die resultierende Konzentration an Emulgator beträgt jeweils 300 ppm.

Zur Inkubation werden die Mikrotiterplatten anschließend 3 bis 5 Tage auf einem Schüttler bei einer Temperatur von 22°C bewegt, bis in der unbehandelten Kontrolle ein ausreichendes Wachstum des jeweiligen Mikroorganismus feststellbar ist.

Die Auswertung erfolgt photometrisch bei einer Wellenlänge von 620 nm. Aus den Messdaten für die verschiedenen Konzentrationen wird die Wirkstoffdosis berechnet, die zu einer 50 %igen Hemmung des Pilzwachstums (ED₅₀) gegenüber der unbehandelten Kontrolle führt.

In diesem Test liegt der ED₅₀-Wert der im Beispiel 1 aufgeführten erfindungsgemäßen Verbindung bei Botrytis cinerea bei einer Wirkstoffdosis, die kleiner als 10 ppm ist.

## Patentansprüche

1. Pyrazolopyrimidine der Formel 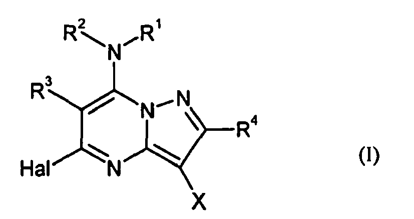 in welcher
*R¹* *für Alkyl mit 1 bis 6 Kohlenstoffatomen steht, das einfach bis fünffach, gleichartig oder verschieden substituiert sein kann durch Halogen, Cyano, Hydroxy, Alkoxy mit 1 bis 4 Kohlenstoffatomen und*/*oder Cycloalkyl mit 3 bis 6 Kohlenstoffatomen, oder*
*R¹* *für Alkenyl mit 2 bis 6 Kohlenstoffatomen steht, das einfach bis dreifach, gleichartig oder verschieden substituiert sein kann durch Halogen, Cyano, Hydroxy, Alkoxy mit 1 bis 4 Kohlenstoffatomen und*/*oder Cycloalkyl mit 3 bis 6 Kohlenstoffatomen, oder*
*R¹* *für Alkinyl mit 3 bis 6 Kohlenstoffatomen steht, das einfach bis dreifach, gleichartig oder verschieden substituiert sein kann durch Halogen, Cyano, Alkoxy mit 1 bis 4 Kohlenstoffatomen und*/*oder Cycloalkyl mit 3 bis 6 Kohlenstoffatomen, oder*
*R¹* *für Cycloalkyl mit 3 bis 6 Kohlenstoffatomen steht, das einfach bis dreifach, gleichartig oder verschieden substituiert sein kann durch Halogen und*/*oder Alkyl mit 1 bis 4 Kohlenstoffatomen, oder*
*R¹* *für gesättigtes oder ungesättigtes Heterocyclyl mit 5 oder 6 Ringgliedern und 1 bis 3 Heteroatomen, wie Stickstoff, Sauerstoff und*/*oder Schwefel, steht, wobei das Heterocyclyl einfach oder zweifach substituiert sein kann durch Halogen, Alkyl mit 1 bis 4 Kohlenstoffatomen, Cyano, Nitro und*/*oder Cycloalkyl mit 3 bis 6 Kohlenstoffatomen,*
R² für Wasserstoff oder Alkyl steht, oder
R¹ und R² gemeinsam mit dem Stickstoffatom, an das sie gebunden sind, für einen gesättigten oder ungesättigten heterocyclischen Ring mit 3 bis 6 Ringgliedern stehen, wobei der Heterocyclus ein weiteres Stickstoff-, Sauerstoff- oder Schwefelatom als Ringglied enthalten kann und wobei der Heterocyclus bis zu dreifach substituiert sein kann durch Fluor, Chlor, Brom, Alkyl mit 1 bis 4 Kohlenstoffatomen und/oder Halogenalkyl mit 1 bis 4 Kohlenstoffatomen und 1 bis 9 Fluor- und/oder Chloratomen,
*R³* *für gesättigtes oder ungesättigtes Heterocyclyl mit 5 oder 6 Ringgliedern und 1 bis 4 Heteroatomen, wie Sauerstoff, Stickstoff und*/*oder Schwefel, steht, wobei das Heterocyclyl einfach bis vierfach, gleichartig oder verschieden substituiert sein kann durch*
*Fluor, Chlor, Brom, Cyano, Nitro, Alkyl, Alkoxy, Hydroximinoalkyl oder Alkoximinoalkyl mit jeweils 1 bis 3 Kohlenstoffatomen pro Alkylteil,*
*Halogenalkyl oder Halogenalkoxy mit jeweils 1 bis 3 Kohlenstoffatomen und 1 bis 7 Halogenatomen,*
R⁴ für Wasserstoff oder Alkyl steht,
Hal für Halogen steht und
X für Halogen, Cyano, Nitro, Alkyl, gegebenenfalls substituiertes Alkenyl, gegebenenfalls substituiertes Alkinyl, Hydroxyalkyl, Alkoxyalkyl, Halogenalkyl, Cycloalkyl, Formyl, Thiocarbamoyl, Alkoxycarbonyl, Alkylcarbonyl, Hydroximinoalkyl, Alkoximinoalkyl, Alkylthio, Alkylsulfinyl, Alkylsulfonyl oder Alkylaminocarbonyl steht.

2. Pyrazolopyrimidin der Formel (I) gemäß Anspruch 1, in denen
R¹ für Alkyl mit 1 bis 6 Kohlenstoffatomen steht, das einfach bis fünffach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen, oder
R¹ für Alkenyl mit 2 bis 6 Kohlenstoffatomen steht, das einfach bis dreifach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen, oder
R¹ für Alkinyl mit 3 bis 6 Kohlenstoffatomen steht, das einfach bis dreifach, gleichartig oder verschieden substituiert sein kann durch Halogen, Cyano, Alkoxy mit 1 bis 4 Kohlenstoffatomen und/oder Cycloalkyl mit 3 bis 6 Kohlenstoffatomen, oder
R¹ für Cycloalkyl mit 3 bis 6 Kohlenstoffatomen steht, das einfach bis dreifach, gleichartig oder verschieden substituiert sein kann durch Halogen und/oder Alkyl mit 1 bis 4 Kohlenstoffatomen, oder
R¹ für gesättigtes oder ungesättigtes Heterocyclyl mit 5 oder 6 Ringgliedern und 1 bis 3 Heteroatomen, wie Stickstoff, Sauerstoff und/oder Schwefel, steht, wobei das Heterocyclyl einfach oder zweifach substituiert sein kann durch Halogen, Alkyl mit 1 bis 4 Kohlenstoffatomen, Cyano, Nitro und/oder Cycloalkyl mit 3 bis 6 Kohlenstoffatomen,
R² für Wasserstoff oder Alkyl mit 1 bis 4 Kohlenstoffatomen steht, oder
R¹ und R² gemeinsam mit dem Stickstoffatom, an das sie gebunden sind, für einen gesättigten oder ungesättigten heterocyclischen Ring mit 3 bis 6 Ringgliedern stehen, wobei der Heterocyclus ein weiteres Stickstoff-, Sauerstoff- oder Schwefelatom als Ringglied enthalten kann und wobei der Heterocyclus bis zu dreifach substituiert sein kann durch Fluor, Chlor, Brom, Alkyl mit 1 bis 4 Kohlenstoffatomen und/oder Halogenalkyl mit 1 bis 4 Kohlenstoffatomen und 1 bis 9 Fluor- und/oder Chloratomen,
R³ für gesättigtes oder ungesättigtes Heterocyclyl mit 5 oder 6 Ringgliedern und 1 bis 4 Heteroatomen, wie Sauerstoff, Stickstoff und/oder Schwefel, steht, wobei das Heterocyclyl einfach bis vierfach, gleichartig oder verschieden substituiert sein kann durch
Fluor, Chlor, Brom, Cyano, Nitro, Alkyl, Alkoxy, Hydroximinoalkyl oder Alkoximinoalkyl mit jeweils 1 bis 3 Kohlenstoffatomen pro Alkylteil,
Halogenalkyl oder Halogenalkoxy mit jeweils 1 bis 3 Kohlenstoffatomen und 1 bis 7 Halogenatomen,
R⁴ für Wasserstoff oder Alkyl mit 1 bis 4 Kohlenstoffatomen steht,
Hal für Fluor, Chlor oder Brom steht und
X für Cyano, Fluor, Chlor, Brom, Iod, Nitro, Formyl, Halogenalkyl mit 1 bis 6 Kohlenstoffatomen und 1 bis 9 Fluor, Chlor und/oder Bromatomen, Alkyl mit 1 bis 4 Kohlenstoffatomen, Alkenyl mit 2 bis 6 Kohlenstoffatomen, durch Carboxy, Methoxycarbonyl oder Ethoxycarbonyl substituiertes Alkenyl mit 2 bis 5 Kohlenstoffatomen im Alkenylteil, Alkinyl mit 2 bis 6 Kohlenstoffatomen, durch Carboxy, Methoxycarbonyl oder Ethoxycarbonyl substituiertes Alkinyl mit 2 bis 5 Kohlenstoffatomen, Hydroxyalkyl mit 1 bis 4 Kohlenstoffatomen, Alkoxyalkyl mit 1 bis 4 Kohlenstoffatomen im Alkoxyteil und 1 bis 4 Kohlenstoffatomen im Alkylteil, Cycloalkyl mit 3 bis 6 Kohlenstoffatomen, Thiocarbamoyl, Alkoxycarbonyl mit 1 bis 4 Kohlenstoffatomen im Alkoxyteil, Alkylcarbonyl mit 1 bis 4 Kohlenstoffatomen im Alkylteil, Hydroximinoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen oder für Alkylaminocarbonyl mit 1 bis 4 Kohlenstoffatomen im Alkylteil steht.

3. Pyrazolopyrimidine der Formel (I) gemäß Anspruch 1 oder 2, in denen
R¹ für einen Rest der Formel 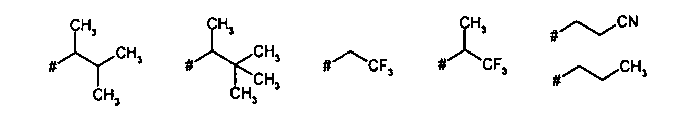 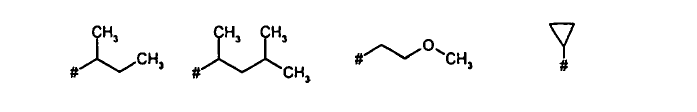 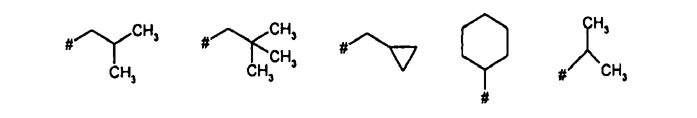 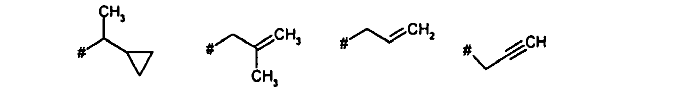 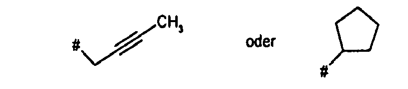 steht,
wobei # die Anknüpfungsstelle markiert,
R² für Wasserstoff, Methyl, Ethyl oder Propyl steht, oder
R¹ und R² gemeinsam mit dem Stickstoffatom, an das sie gebunden sind, für Pyrrolidinyl, Piperidinyl, Morpholinyl, Thiomorpholinyl, Piperazinyl, 3,6-Dihydro-1(2H)-piperidinyl oder Tetrahydro-1(2H)-pyridazinyl stehen, wobei diese Reste durch 1 bis 3 Fluoratome, 1 bis 3 Methylgruppen und/oder Trifluormethyl substituiert sein können,
oder
R¹ und R² gemeinsam mit dem Stickstoffatom, an das sie gebunden sind, für einen Rest der Formel 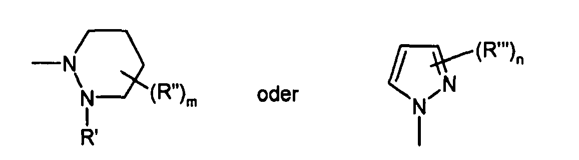 worin
R' für Wasserstoff oder Methyl steht,
R" für Methyl, Ethyl, Fluor, Chlor oder Trifluormethyl steht,
m für die Zahlen 0, 1, 2 oder 3 steht, wobei R" für gleiche oder verschiedene Reste steht, wenn m für 2 oder 3 steht,
R"' für Methyl, Ethyl, Fluor, Chlor oder Trifluormethyl steht
und
n für die Zahlen 0, 1, 2 oder 3 steht, wobei R"' für gleiche oder verschiedene Reste steht, wenn n für 2 oder 3 steht,
R³ für Pyridyl steht, das in 2- oder 4-Stellung verknüpft ist und einfach bis vierfach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinomethyl, Methoximinoethyl und/oder Trifluormethyl, oder
R³ für Pyrimidyl steht, das in 2- oder 4-Stellung verknüpft ist und einfach bis dreifach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinomethyl, Methoximinoethyl und/oder Trifluormethyl, oder
R³ für Thienyl steht, das in 2- oder 3-Stellung verknüpft ist und einfach bis dreifach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinimethyl, Methoximinoethyl und/oder Trifluormethyl, oder
R³ für Thiazolyl steht, das in 2-, 4- oder 5-Stellung verknüpft ist und einfach bis zweifach, gleichartig oder verschieden substituiert sein kann durch Fluor, Chlor, Brom, Cyano, Nitro, Methyl, Ethyl, Methoxy, Methylthio, Hydroximinomethyl, Hydroximinoethyl, Methoximinimethyl, Methoximinoethyl und/oder Trifluormethyl,
R⁴ für Wasserstoff, Methyl, Ethyl, Propyl oder Isopropyl steht,
Hal für Fluor oder Chlor steht und
X für Cyano, Fluor, Chlor, Brom, Jod, Nitro, Formyl, Trifluormethyl, Difluormethyl, Methyl, Ethyl, Cyclopropyl, Thiocarbamoyl, Methoxycarbonyl, Methylcarbonyl, Ethylcarbonyl, Hydroximinomethyl, Methoximinomethyl, Methylthio, Methylsulfinyl Methylsulfonyl, Methylaminocarbonyl, Hydroxymethyl, Hydroxyeth-1-yl, Methoxymethyl, Ethoxymethyl oder 1-Methoxy-ethyl steht, oder
X für einen Rest der Formel

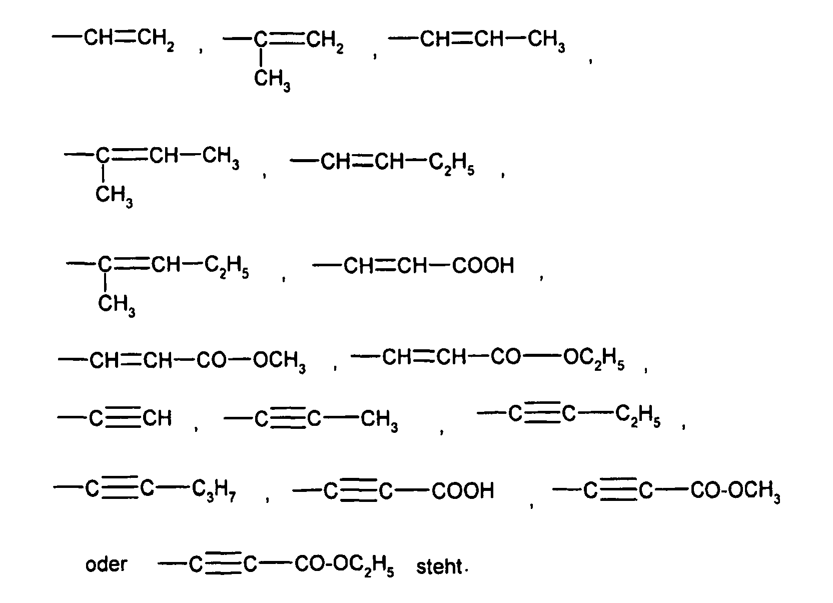

4. Verfahren zur Herstellung von Pyrazolopyrimidinen der Formel (I) gemäß Anspruch 1, **dadurch gekennzeichnet, dass** man
(a) Halogen-pyrazolopyrimidine der Formel 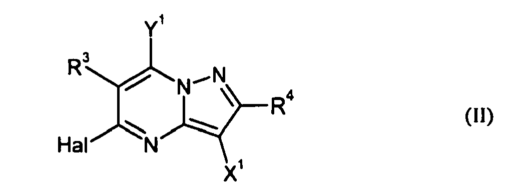 in welcher
R³, R⁴ und Hal die im Anspruch 1 angegebenen Bedeutungen haben,
X¹ für Halogen, Cyano, Nitro, Alkyl, Halogenalkyl, Cycloalkyl, Formyl, Thiocarbamoyl, Alkoxycarbonyl, Alkylcarbonyl, Alkylthio, Alkylsulfinyl, Alkylsulfonyl oder Alkylaminocarbonyl steht und
Y¹ für Halogen steht,
mit Aminen der Formel 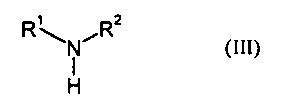 in welcher
R¹ und R² die im Anspruch 1 angegebenen Bedeutungen haben,
gegebenenfalls in Gegenwart eines Verdünnungsmittels, gegebenenfalls in Gegenwart eines Säureakzeptors und gegebenenfalls in Gegenwart eines Katalysators umsetzt, oder
b) Pyrazolopyrimidine der Formel 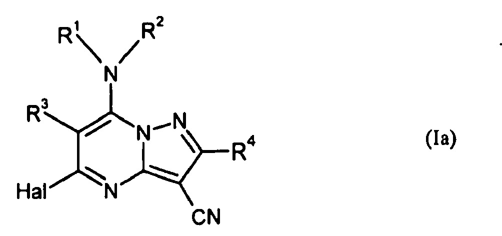 in welcher
R¹, R², R³, R⁴ und Hal die im Anspruch 1 angegebenen Bedeutungen haben,
entweder
α) mit Diisobutyl-aluminiumhydrid in Gegenwart von wässriger Ammoniumchlorid-Lösung sowie in Gegenwart eines organischen Verdünnungsmittels umsetzt,
oder
β) mit Grignard-Verbindungen der Formel
R⁵ - Mg - X² (IV)
in welcher
R⁵ für Alkyl steht und
X² für Chlor oder Brom steht,
in Gegenwart eines Verdünnungsmittels und gegebenenfalls in Gegenwart eines Katalysators umsetzt,
oder
c) Pyrazolopyrimidine der Formel 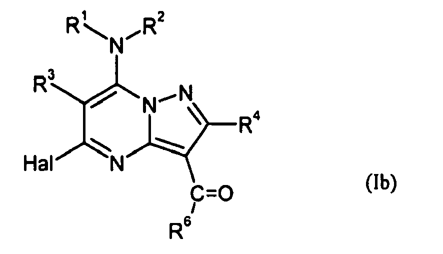 in welcher
R¹, R², R³, R⁴ und Hal die oben angegebenen Bedeutungen haben und
R⁶ für Wasserstoffoder Alkyl steht,
entweder
α) mit Amino-Verbindungen der Formel
H₂N-OR⁷ (V)
in welcher
R⁷ für Wasserstoff oder Alkyl steht,
in Gegenwart eines Verdünnungsmittels und gegebenenfalls in Gegenwart eines Katalysators umsetzt, wobei die Amino-Verbindungen der Formel (V) auch in Form ihrer Säureadditions-Salze eingesetzt werden können, oder
β) mit Triphenylphosphonium-Salzen der Formel 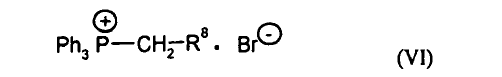
in welcher
Ph für Phenyl steht und
R⁸ für Wasserstoff oder gegebenenfalls substituiertes Alkyl steht,
in Gegenwart einer Base sowie in Gegenwart eines Verdünnungsmittels umsetzt,
oder
γ) mit Diisobutyl-aluminiumhydrid in Gegenwart von wässriger Ammoniumchlorid-Lösung sowie in Gegenwart eines organischen Verdünnungsmittels umsetzt,
oder mit Natriumborhydrid in Gegenwart eines Verdünnungsmittels umsetzt,
und gegebenenfalls die dabei entstehenden Pyrazolopyrimidine der Formel 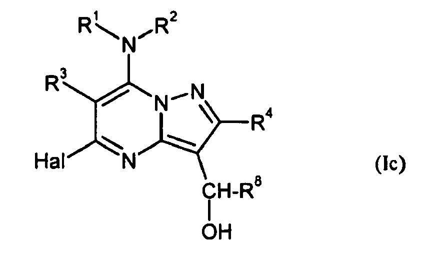 in welcher
R¹, R², R³, R⁸ und Hal die oben angegebenen Bedeutungen haben,
mit Alkylierungsmitteln der Formel
R⁹ - X³ (VII)
in welcher
R⁹ für Alkyl steht und
X³ für Chlor, Brom, Iod oder den Rest R⁹O-SO₂-O- steht,
gegebenenfalls in Gegenwart einer Base und in Gegenwart eines Verdünnungsmittels umsetzt,
oder
d) Pyrazolopyrimidine der Formel 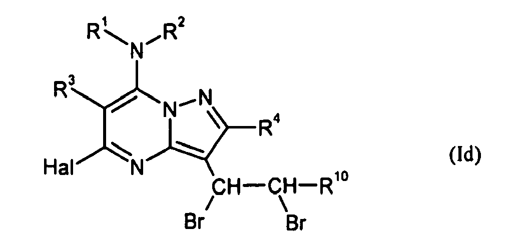 in welcher
R¹, R², R³, R⁴ und Hal die oben angegebenen Bedeutungen haben,
R¹⁰ für Wasserstoff oder gegebenenfalls substituiertes Alkyl steht,
mit starken Basen in Gegenwart eines Verdünnungsmittels umsetzt, oder
e) Pyrazolopyrimidine der Formel 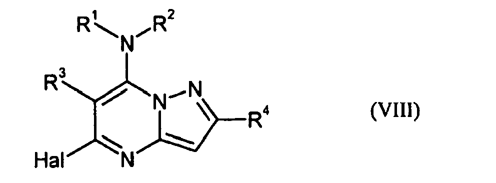 in welcher
R¹, R², R³, R⁴ und Hal die oben angegebenen Bedeutungen haben,
mit Acyl-Derivaten der Formel 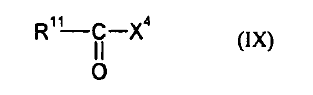 in welcher
R¹¹ für Alkyl steht und
X⁴ für Chlor oder einen Rest der Formel 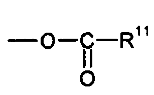 steht,
in Gegenwart eines Katalysators und in Gegenwart eines Verdünnungsmittels umsetzt.

5. Mittel zur Bekämpfung von unerwünschten Mikroorganismen, **gekennzeichnet durch** einen Gehalt an mindestens einem Pyrazolopyrimidin der Formel (I) gemäß einem oder mehreren der Ansprüche 1 bis 3 neben Streckmitteln und/oder oberflächenaktiven Stoffen.

6. Verwendung von Pyrazolopyrimidinen der Formel (I) gemäß einem oder mehreren der Ansprüche 1 bis 3 *zur Herstellung eines Mittels* zur Bekämpfung von unerwünschten Mikroorganismen.

7. Verfahren zur Bekämpfung von unerwünschten Mikroorganismen *im Pflanzenschutz und im Materialschutz,* **dadurch gekennzeichnet, dass** man Pyrazolopyrimidine der Formel (I) gemäß einem oder mehreren der Ansprüche 1 bis 3 auf die unerwünschten Mikroorganismen und/oder deren Lebensraum ausbringt.

8. Verfahren zur Herstellung von Mitteln zur Bekämpfung von unerwünschten Mikroorganismen, **dadurch gekennzeichnet, dass** man Pyrazolopyrimidine der Formel (I) gemäß einem oder mehreren der Ansprüche 1 bis 3 mit Streckmitteln und/oder oberflächenaktiven Stoffen vermischt.

9. Halogen-pyrazolopyrimidine der Formel 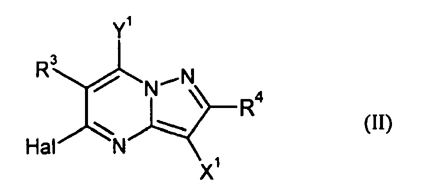 in welcher
*R³* *für gesättigtes oder ungesättigtes Heterocyclyl mit 5 oder 6 Ringgliedern und 1 bis 4 Heteroatomen, wie Sauerstoff, Stickstoff und*/*oder Schwefel, steht, wobei das Heterocyclyl einfach bis vierfach, gleichartig oder verschieden substituiert sein kann durch*
*Fluor, Chlor, Brom, Cyano, Nitro, Alkyl, Alkoxy, Hydroximinoalkyl oder Alkoximinoalkyl mit jeweils 1 bis 3 Kohlenstoffatomen pro Alkylteil,*
*Halogenalkyl oder Halogenalkoxy mit jeweils 1 bis 3 Kohlenstoffatomen und 1 bis 7 Halogenatomen,*
R⁴ für Wasserstoff oder Alkyl steht,
Hal für Halogen steht,
X¹ für Halogen, Cyano, Nitro, Alkyl, Halogenalkyl, Cycloalkyl, Formyl, Thiocarbamoyl, Alkoxycarbonyl, Alkylcarbonyl, Alkylthio, Alkylsulfinyl, Alkylsulfonyl oder Alkylaminocarbonyl steht und
Y¹ für Halogen steht.

10. Verfahren zur Herstellung von Halogen-pyrazolopyrimidinen der Formel (II) gemäß Anspruch 9, **dadurch gekennzeichnet, dass** man
f) Hydroxy-pyrazolopyrimidine der Formel 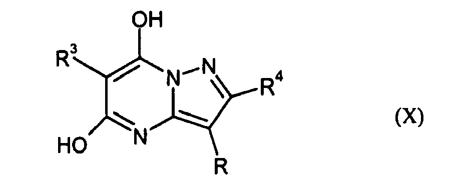 in welcher
R³ und R⁴ die im Anspruch 9 angegebenen Bedeutungen haben, und
R für Halogen, Cyano, Nitro, Alkyl, Halogenalkyl, Cycloalkyl, Thiocarbamoyl, Alkoxycarbonyl, Alkylthio, Alkylsulfinyl, Alkylsulfonyl oder Alkylaminocarbonyl steht,
mit Halogenierungsmitteln, gegebenenfalls in Gegenwart eines Verdünnungsmittels umsetzt,
oder
g) Hydroxy-pyrazolopyrimidine der Formel 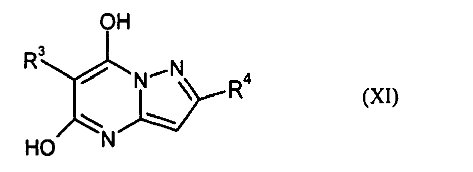 in welcher
R³ und R⁴ die im Anspruch 9 angegebenen Bedeutungen haben,
mit Phosphoroxychlorid in Gegenwart von Dimethylformamid umsetzt und gegebenenfalls unter Zugabe von Phosphorpentachlorid nachreagieren lässt.

11. Hydroxy-pyrazolopyrimidine der Formel 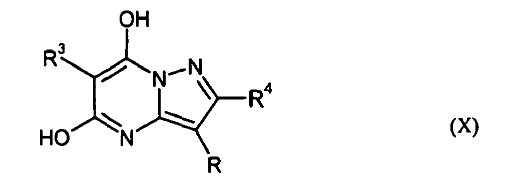 in welcher
*R³* *für gesättigtes oder ungesättigtes Heterocyclyl mit 5 oder 6 Ringgliedern und 1 bis 4 Heteroatomen, wie Sauerstoff, Stickstoff und*/*oder Schwefel, steht, wobei das Heterocyclyl einfach bis vierfach, gleichartig oder verschieden substituiert sein kann durch*
*Fluor, Chlor, Brom, Cyano, Nitro, Alkyl, Alkoxy, Hydroximinoalkyl oder Alkoximinoalkyl mit jeweils 1 bis 3 Kohlenstoffatomen pro Alkylteil,*
*Halogenalkyl oder Halogenalkoxy mit jeweils 1 bis 3 Kohlenstoffatomen und 1 bis 7 Halogenatomen,*
R⁴ für Wasserstoff oder Alkyl steht und
R für Halogen, Cyano, Nitro, Alkyl, Halogenalkyl, Cycloalkyl, Thiocarbamoyl, Alkoxycarbonyl, Alkylthio, Alkylsulfinyl, Alkylsulfonyl oder Alkylaminocarbonyl steht.

12. Verfahren zur Herstellung von Hydroxy-pyrazolopyrimidinen der Formel (X) gemäß Anspruch 11, **dadurch gekennzeichnet, dass** man
(h) Heterocyclylmalonester der Formel 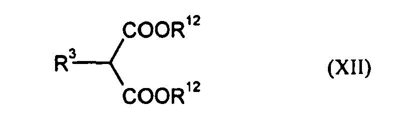 in welcher
R³ die im Anspruch 11 angegebene Bedeutung hat und
R¹² für Alkyl mit 1 bis 4 Kohlenstoffatomen steht,
mit Aminopyrazolen der Formel 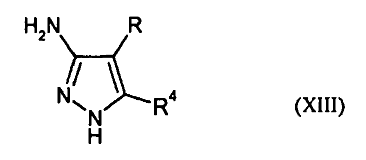 in welcher
R⁴ und R die im Anspruch 11 angegebenen Bedeutungen haben,
gegebenenfalls in Gegenwart eines Verdünnungsmittels und gegebenenfalls in Gegenwart eines Säurebindemittels umsetzt.

## Claims

1. Pyrazolopyrimidines of the formula 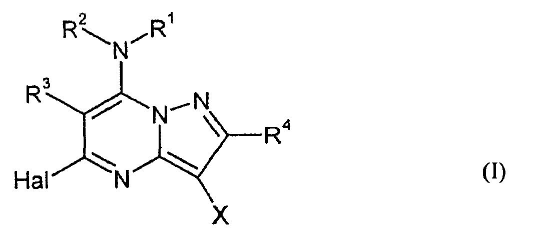 in which
R¹ represents alkyl having 1 to 6 carbon atoms which may be mono- to pentasubstituted by identical or different substituents from the group consisting of halogen, cyano, hydroxy, alkoxy having 1 to 4 carbon atoms and cycloalkyl having 3 to 6 carbon atoms, or
R¹ represents alkenyl having 2 to 6 carbon atoms which may be mono- to trisubstituted by identical or different substituents from the group consisting of halogen, cyano, hydroxy, alkoxy having 1 to 4 carbon atoms and cycloalkyl having 3 to 6 carbon atoms, or
R¹ represents alkinyl having 3 to 6 carbon atoms which may be mono- to trisubstituted by identical or different substituents from the group consisting of halogen, cyano, alkoxy having 1 to 4 carbon atoms and cycloalkyl having 3 to 6 carbon atoms, or
R¹ represents cycloalkyl having 3 to 6 carbon atoms which may be mono- to trisubstituted by identical or different substituents from the group consisting of halogen and/or alkyl having 1 to 4 carbon atoms, or
R¹ represents saturated or unsaturated heterocyclyl having 5 or 6 ring members and 1 to 3 heteroatoms, such as nitrogen, oxygen and/or sulphur, where the heterocyclyl may be mono- or disubstituted by halogen, alkyl having 1 to 4 carbon atoms, cyano, nitro and/or cycloalkyl having 3 to 6 carbon atoms,
R² represents hydrogen or alkyl, or
R¹ and R² together with the nitrogen atom to which they are attached represent a saturated or unsaturated heterocyclic ring having 3 to 6 ring members, where the heterocycle may contain a further nitrogen, oxygen or sulphur atom as ring member and where the heterocycle may be substituted up to 3 times by fluorine, chlorine, bromine, alkyl having 1 to 4 carbon atoms and/or haloalkyl having 1 to 4 carbon atoms and 1 to 9 fluorine and/or chlorine atoms,
R³ represents saturated or unsaturated heterocyclyl having 5 or 6 ring members and 1 to 4 heteroatoms, such as oxygen, nitrogen and/or sulphur, where the heterocyclyl may be mono- to tetrasubstituted by identical or different substituents from the group consisting of
fluorine, chlorine, bromine, cyano, nitro, alkyl, alkoxy, hydroximinoalkyl and alkoximinoalkyl having in each case 1 to 3 carbon atoms per alkyl moiety,
haloalkyl or haloalkoxy having in each case 1 to 3 carbon atoms and 1 to 7 halogen atoms,
R⁴ represents hydrogen or alkyl,
Hal represents halogen and
X represents halogen, cyano, nitro, alkyl, optionally substituted alkenyl, optionally substituted alkinyl, hydroxyalkyl, alkoxyalkyl, haloalkyl, cycloalkyl, formyl, thiocarbamoyl, alkoxycarbonyl, alkylcarbonyl, hydroximinoalkyl, alkoximinoalkyl, alkylthio, alkylsulphinyl, alkylsulphonyl or alkylaminocarbonyl.

2. Pyrazolopyrimidine of the formula (I) according to Claim 1 in which
R¹ represents alkyl having 1 to 6 carbon atoms which may be mono- to pentasubstituted by identical or different substituents from the group consisting of halogen, cyano, hydroxy, alkoxy having 1 to 4 carbon atoms and cycloalkyl having 3 to 6 carbon atoms, or
R¹ represents alkenyl having 2 to 6 carbon atoms which may be mono- to trisubstituted by identical or different substituents from the group consisting of halogen, cyano, hydroxy, alkoxy having I to 4 carbon atoms and cycloalkyl having 3 to 6 carbon atoms, or
R¹ represents alkinyl having 3 to 6 carbon atoms which may be mono- to trisubstituted by identical or different substituents from the group consisting of halogen, cyano, alkoxy having 1 to 4 carbon atoms and cycloalkyl having 3 to 6 carbon atoms, or
R¹ represents cycloalkyl having 3 to 6 carbon atoms which may be mono- to trisubstituted by identical or different substituents from the group consisting of halogen and/or alkyl having 1 to 4 carbon atoms, or
R¹ represents saturated or unsaturated heterocyclyl having 5 or 6 ring members and 1 to 3 heteroatoms, such as nitrogen, oxygen and/or sulphur, where the heterocyclyl may be mono- or disubstituted by halogen, alkyl having 1 to 4 carbon atoms, cyano, nitro and/or cycloalkyl having 3 to 6 carbon atoms,
R² represents hydrogen or alkyl having 1 to 4 carbon atoms, or
R¹ and R² together with the nitrogen atom to which they are attached represent a saturated or unsaturated heterocyclic ring having 3 to 6 ring members, where the heterocycle may contain a further nitrogen, oxygen or sulphur atom as ring member and where the heterocycle may be substituted up to 3 times by fluorine, chlorine, bromine, alkyl having 1 to 4 carbon atoms and/or haloalkyl having 1 to 4 carbon atoms and 1 to 9 fluorine and/or chlorine atoms,
R³ represents saturated or unsaturated heteocyclyl having 5 or 6 ring members and 1 to 4 heteroatoms, such as oxygen, nitrogen and/or sulphur, where the heterocyclyl may be mono- or tetrasubstituted by identical or different substituents from the group consisting of fluorine, chlorine, bromine, cyano, nitro, alkyl, alkoxy, hydroximinoalkyl and alkoximinoalkyl having in each case 1 to 3 carbon atoms per alkyl moiety,
haloalkyl or haloalkoxy having in each case 1 to 3 carbon atoms and 1 to 7 halogen atoms,
R⁴ represents hydrogen or alkyl having 1 to 4 carbon atoms,
Hal represents fluorine, chlorine or bromine and
X represents cyano, fluorine, chlorine, bromine, iodine, nitro, formyl, haloalkyl having 1 to 6 carbon atoms and 1 to 9 fluorine, chlorine and/or bromine atoms, alkyl having 1 to 4 carbon atoms, alkenyl having 2 to 6 carbon atoms, carboxy-, methoxycarbonyl- or ethoxycarbonyl-substituted alkenyl having 2 to 5 carbon atoms in the alkenyl moiety, alkinyl having 2 to 6 carbon atoms, carboxy-, methoxycarbonyl- or ethoxycarbonyl-substituted alkinyl having 2 to 5 carbon atoms, hydroxyalkyl having 1 to 4 carbon atoms, alkoxyalkyl having 1 to 4 carbon atoms in the alkoxy moiety and 1 to 4 carbon atoms in the alkyl moiety, cycloalkyl having 3 to 6 carbon atoms, thiocarbomoyl, alkoxycarbonyl having 1 to 4 carbon atoms in the alkoxy moiety, alkylcarbonyl having 1 to 4 carbon atoms in the alkyl moiety, hydroximinoalkyl having 1 to 4 carbon atoms in the alkyl moiety, alkoximinoalkyl having 1 to 4 carbon atoms in the alkoxy moiety and 1 to 4 carbon atoms in the alkyl moiety, alkylthio having 1 to 4 carbon atoms, alkylsulphinyl having 1 to 4 carbon atoms, alkylsulphonyl having 1 to 4 carbon atoms or represents alkylaminocarbonyl having 1 to 4 carbon atoms in the alkyl moiety.

3. Pyrazolopyrimidines of the formula (I) according to Claim 1 or 2 in which
R¹ represents a radical of the formula 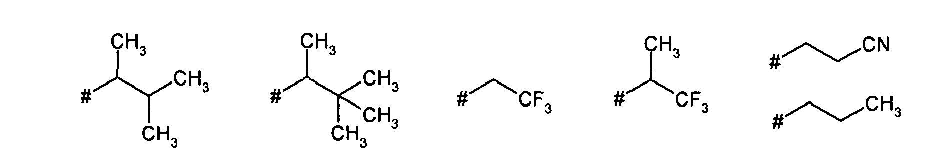 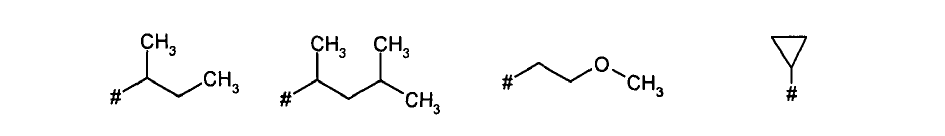 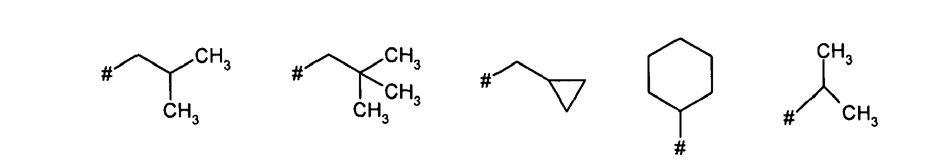 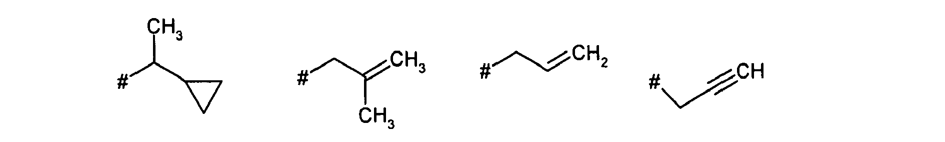 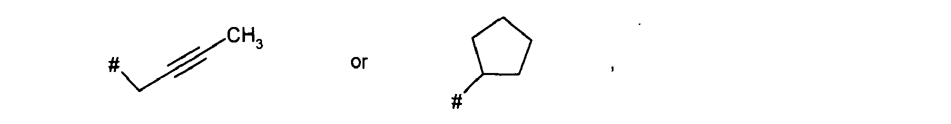
where # denotes the point of attachment,
R² represents hydrogen, methyl, ethyl or propyl, or
R¹ and R² together with the nitrogen atom to which they are attached represent pyrrolidinyl, piperidinyl, morpholinyl, thiomorpholinyl, piperazinyl, 3,6-dihydro-1(2H)-piperidinyl or tetrahydro-1(2H)-pyridazinyl, where these radicals may be substituted by 1 to 3 fluorine atoms, 1 to 3 methyl groups and/or trifluoromethyl,
or
R¹ and R² together with the nitrogen atom to which they are attached represent a radical of the formula 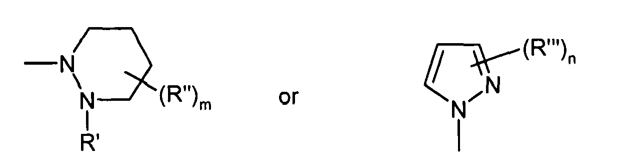 in which
R' represents hydrogen or methyl,
R" represents methyl, ethyl, fluorine, chlorine or trifluoromethyl,
m represents the numbers 0, 1, 2 or 3, where R" represents identical or different radicals, if m represents 2 or 3,
R"' represents methyl, ethyl, fluorine, chlorine or trifluoromethyl
and
n represents the numbers 0, 1, 2 or 3, where R"' represents identical or different radicals if n represents 2 or 3,
R³ represents pyridyl which is attached in the 2- or 4-position and may be mono- to tetrasubstituted by identical or different substituents from the group consisting of fluorine, chlorine, bromine, cyano, nitro, methyl, ethyl, methoxy, methylthio, hydroximinomethyl, hydroximinoethyl, methoximinomethyl, methoximinoethyl and trifluoromethyl, or
R³ represents pyrimidyl which is attached in the 2- or 4-position and may be mono- to trisubstituted by identical or different substituents from the group consisting of fluorine, chlorine, bromine, cyano, nitro, methyl, ethyl, methoxy, methylthio, hydroximinomethyl, hydroximinoethyl, methoximinomethyl, methoximinoethyl and trifluoromethyl, or
R³ represents thienyl which is attached in the 2- or 3-position and may be mono- to trisubstituted by identical or different substituents from the group consisting of fluorine, chlorine, bromine, cyano, nitro, methyl, ethyl, methoxy, methylthio, hydroximinomethyl, hydroximinoethyl, methoximinomethyl, methoximinoethyl and trifluoromethyl, or
R³ represents thiazolyl which is attached in the 2-, 4- or 5-position and which may be mono- to disubstituted by identical or different substituents from the group consisting of fluorine, chlorine, bromine, cyano, nitro, methyl, ethyl, methoxy, methylthio, hydroximinomethyl, hydroximinoethyl, methoximinomethyl, methoximinoethyl and trifluoromethyl,
R⁴ represents hydrogen, methyl, ethyl, propyl or isopropyl,
Hal represents fluorine or chlorine and
X represents cyano, fluorine, chlorine, bromine, iodine, nitro, formyl, trifluoromethyl, difluoromethyl, methyl, ethyl, cyclopropyl, thiocarbamoyl, methoxycarbonyl, methylcarbonyl, ethylcarbonyl, hydroximinomethyl, methoximinomethyl, methylthio, methylsulphinyl, methylsulphonyl, methylaminocarbonyl, hydroxymethyl, hydroxyeth-1-yl, methoxymethyl, ethoxymethyl or 1-methoxyethyl, or
X represents a radical of the formula

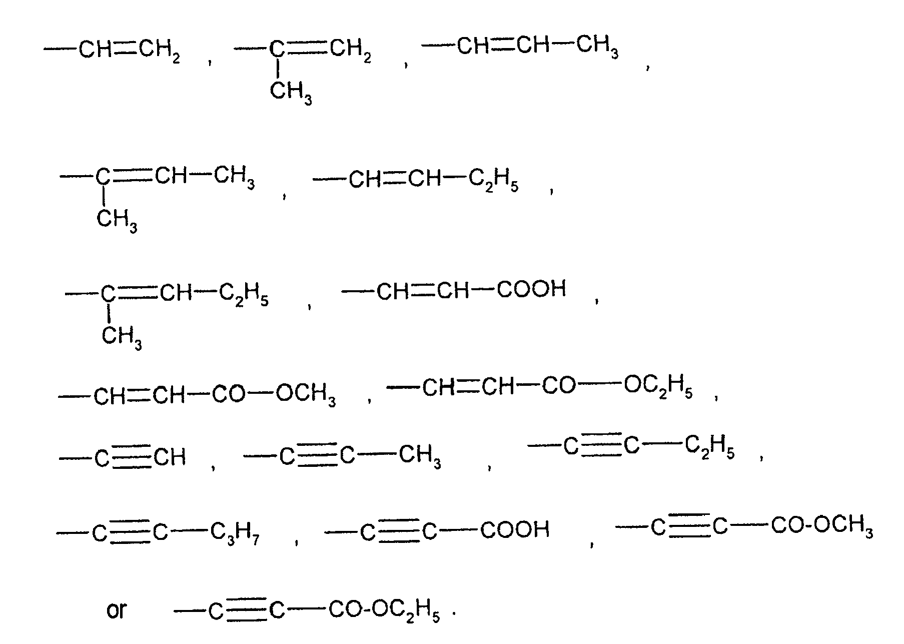

4. Process for preparing pyrazolopyrimidines of the formula (I) according to Claim 1,
**characterized in that**
(a) halopyrazolopyrimidines of the formula 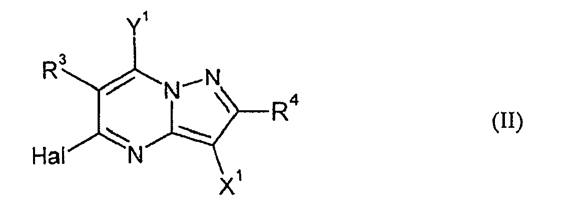 in which
R³, R⁴ and Hal are as defined in Claim 1,
X¹ represents halogen, cyano, nitro, alkyl, haloalkyl, cycloalkyl, formyl, thiocarbamoyl, alkoxycarbonyl, alkylcarbonyl, alkylthio, alkylsulphinyl, alkylsulphonyl or alkylaminocarbonyl and
Y¹ represents halogen,
are reacted with amines of the formula 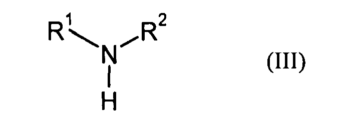 in which
R¹ and R² are as defined in Claim 1,
if appropriate in the presence of a diluent, if appropriate in the presence of an acidic receptor and if appropriate in the presence of a catalyst,
or
b) pyrazolopyrimidines of the formula 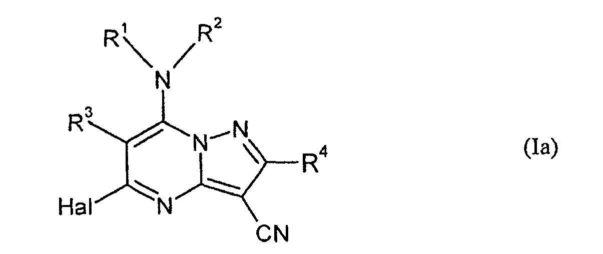 in which
R¹, R², R³, R⁴ and Hal are as defined in Claim 1 are either
α) reacted with diisobutylaluminum hydride in the presence of aqueous ammonium chloride solution and in the presence of an organic diluent,
or
β) reacted with Grignard compounds of the formula
R⁵ - Mg - X² (IV)
in which
R⁵ represents alkyl and
X² represents chlorine or bromine,
in the presence of a diluent and, if appropriate, in the presence of a catalyst,
or
c) pyrazolopyrimidines of the formula 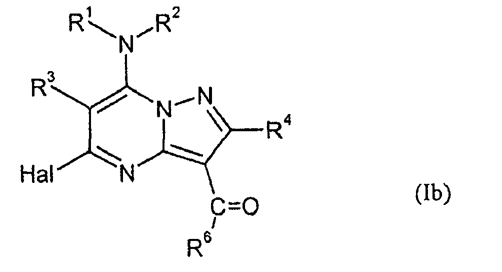 in which
R¹, R², R³, R⁴ and Hal are as defined above and
R⁶ represents hydrogen or alkyl,
are either
α) reacted with amino compounds of the formula
H₂N-OR⁷ (V)
in which
R⁷ represents hydrogen or alkyl,
in the presence of a diluent and, if appropriate, in the presence of a catalyst, where the amino compounds of the formula (V) can also be employed in the form of their acid addition salts,
or
β) reacted with triphenylphosphonium salts of the formula 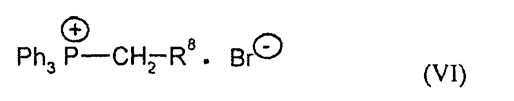 in which
Ph represents phenyl and
R⁸ represents hydrogen or optionally substituted alkyl,
in the presence of a base and in the presence of a diluent,
or
γ) reacted with diisobutylaluminum hydride in the presence of aqueous ammonium chloride solution and in the presence of an organic diluent,
or reacted with sodium borohydride in the presence of a diluent,
and the resulting pyrazolopyrimidines of the formula 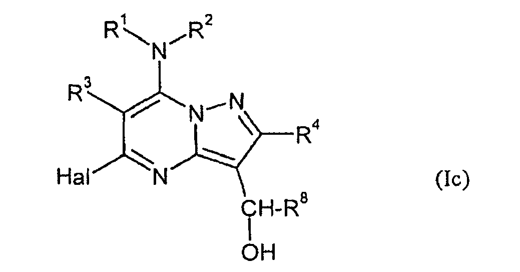 in which
R¹, R², R³, R⁸ and Hal are as defined above
are, if appropriate, reacted with alkylating agents of the formula
R⁹ - X³ (VII)
in which
R⁹ represents alkyl and
X³ represents chlorine, bromine, iodine or the radical R⁹O-SO₂-O-,
if appropriate in the presence of a base and in the presence of a diluent,
or
d) pyrazolopyrimidines of the formula 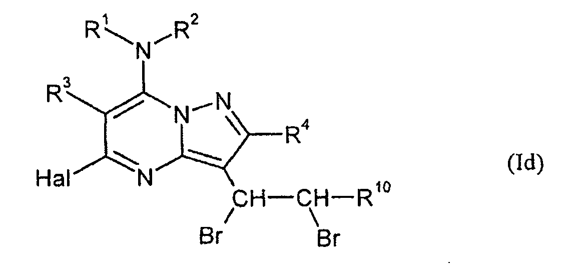 in which
R¹, R², R³, R⁴ and Hal are as defined above,
R¹⁰ represents hydrogen or optionally substituted alkyl,
are reacted with strong bases in the presence of a diluent,
or
e) pyrazolopyrimidines of the formula 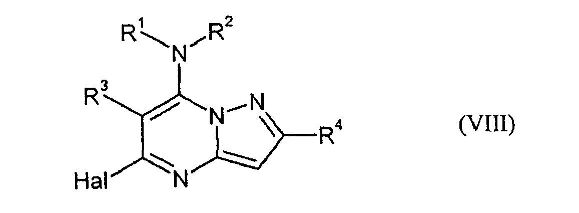 in which
R¹, R², R³, R⁴ and Hal are as defined above are reacted with acyl derivatives of the formula 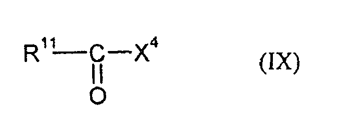 in which
R¹¹ represents alkyl and
X⁴ represents chlorine or a radical of the formula 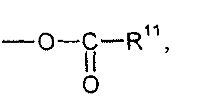
in the presence of a catalyst and in the presence of a diluent.

5. Composition for controlling unwanted microorganisms, **characterized in that** it comprises at least one pyrazolopyrimidine of the formula (I) according to one or more of Claims 1 to 3, in addition to extenders and/or surfactants.

6. Use of pyrazolopyrimidines of the formula (I) according to one or more of Claims 1 to 3 for preparing a composition for controlling unwanted microorganisms.

7. Method for controlling unwanted microorganisms in crop protection and in the protection of materials, **characterized in that** pyrazolopyrimidines of the formula (I) according to one or more of claims 1 to 3 are applied to the unwanted microorganisms and/or their habitat.

8. A process for preparing compositions for controlling unwanted microorganisms, **characterized in that** pyrazolopyrimidines of the formula (I) according to one or more of Claims 1 to 3 are mixed with extenders and/or surfactants.

9. Halopyrazolopyrimidines of the formula 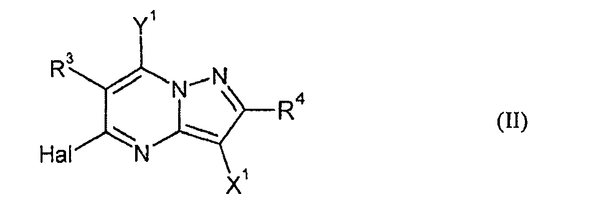 in which
R³ represents saturated or unsaturated heterocyclyl having 5 or 6 ring members and 1 to 4 heteroatoms, such as oxygen, nitrogen and/or sulphur where the heterocyclyl may be mono- to tetrasubstituted by identical or different substituents from the group consisting of
fluorine, chlorine, bromine, cyano, nitro, alkyl, alkoxy, hydroximinoalkyl and alkoximinoalkyl having in each case 1 to 3 carbon atoms per alkyl moiety,
haloalkyl or haloalkoxy having in each case 1 to 3 carbon atoms and 1 to 7 halogen atoms,
R⁴ represents hydrogen or alkyl,
Hal represents halogen,
X¹ represents halogen, cyano, nitro, alkyl, haloalkyl, cycloalkyl, formyl, thiocarbamoyl, alkoxycarbonyl, alkylcarbonyl, alkylthio, alkylsulphinyl, alkylsulphonyl or alkylaminocarbonyl and
Y¹ represents halogen.

10. Process for preparing halopyrazolopyrimidines of the formula (II) according to Claim 9,
**characterized in that**
f) hydroxypyrazolopyrimidines of the formula 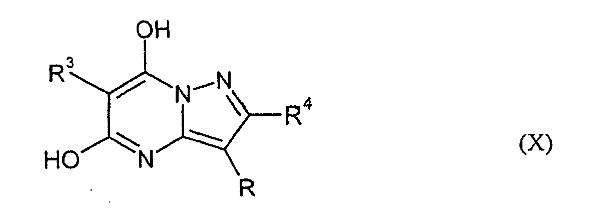 in which
R³ and R⁴ have the meanings in Claim 9, and
R represents halogen, cyano, nitro, alkyl, haloalkyl, cycloalkyl, thiocarbamoyl, alkoxycarbonyl, alkylthio, alkylsulphinyl, alkylsulphonyl or alkylaminocarbonyl
are reacted with halogenating agents, if appropriate in the presence of a diluent,
or
g) hydroxypyrazolopyrimidines of the formula 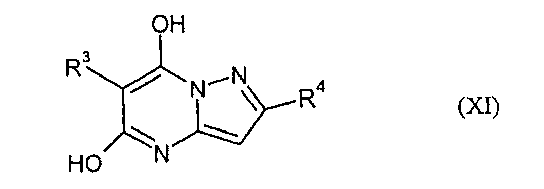
in which
R³ and R⁴ have the meanings given in Claim 9
are reacted with phosphorus oxychloride in the presence of dimethylformamide and, if appropriate, reacted further with addition of phosphorus pentachloride.

11. Hydroxypyrazolopyrimidines of the formula 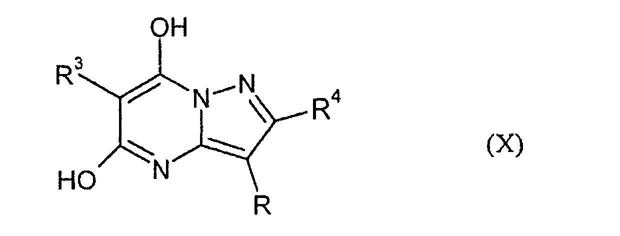 in which
R³ represents saturated or unsaturated heterocyclyl having 5 or 6 ring members and 1 to 4 heteroatoms, such as oxygen, nitrogen and/or sulphur where the heterocyclyl may be mono- to tetrasubstituted by identical or different substituents from the group consisting of
fluorine, chlorine, bromine, cyano, nitro, alkyl, alkoxy, hydroximinoalkyl and alkoximinoalkyl having in each case 1 to 3 carbon atoms per alkyl moiety,
haloalkyl or haloalkoxy having in each case 1 to 3 carbon atoms and 1 to 7 halogen atoms,
R⁴ represents hydrogen or alkyl and
R represents halogen, cyano, nitro, alkyl, haloalkyl, cycloalkyl, thiocarbamoyl, alkoxycarbonyl, alkylthio, alkylsulphinyl, alkylsulphonyl or alkylaminocarbonyl.

12. Process for preparing hydroxypyrazolopyrimidines of the formula (X) according to Claim 11, **characterized in that**
(h) heteocyclylmalonic esters of the formula 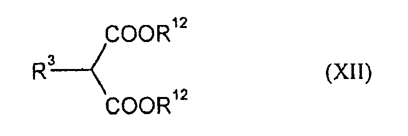 in which
R³ has the meaning given in Claim 11 and
R¹² represents alkyl having 1 to 4 carbon atoms,
are reacted with aminopyrazoles of the formula 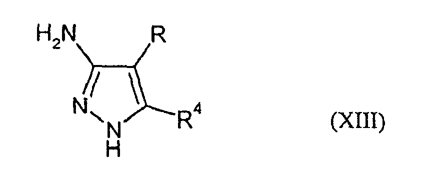 in which
R⁴ and R have the meanings given in Claim 11,
if appropriate in the presence of a diluent and if appropriate in the presence of an acid binder.

## Revendications

1. Pyrazolopyrimidines de la formule : 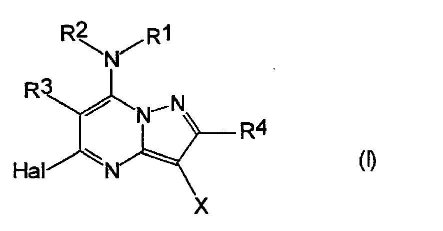 dans laquelle
R¹ représente un radical alcoyle ayant 1 à 6 atomes de carbone, qui peut être substitué une à cinq fois, de manière identique ou différente, par un atome d'halogène, un radical cyano, hydroxy, alcoxy ayant 1 à 4 atomes de carbone et/ou cycloalcoyle ayant 3 à 6 atomes de carbone, ou
R¹ représente un radical alcényle ayant 2 à 6 atomes de carbone, qui peut être substitué une à trois fois, de manière identique ou différente, par un atome d'halogène, un radical cyano, hydroxy, alcoxy ayant 1 à 4 atomes de carbone et/ou cycloalcoyle ayant 3 à 6 atomes de carbone, ou
R¹ représente un radical alcynyle ayant 3 à 6 atomes de carbone, qui peut être substitué une à trois fois, de manière identique ou différente, par un atome d'halogène, un radical cyano, alcoxy ayant 1 à 4 atomes de carbone et/ou cycloalcoyle ayant 3 à 6 atomes de carbone, ou
R¹ représente un radical cycloalcoyle ayant 3 à 6 atomes de carbone, qui peut être substitué une à trois fois, de manière identique ou différente, par un atome d'halogène et/ou un radical alcoyle ayant 1 à 4 atomes de carbone, ou
R¹ représente un radical hétérocyclyle saturé ou insaturé ayant 5 ou 6 membres cycliques et 1 à 3 hétéroatomes, comme l'azote, l'oxygène et/ou le soufre, où le radical hétérocyclyle peut être substitué un ou deux fois, par un atome d'halogène, un radical alcoyle ayant 1 à 4 atomes de carbone, un radical cyano, nitro et/ou cycloalcoyle ayant 3 à 6 atomes de carbone,
R² représente l'atome d'hydrogène ou un radical alcoyle, ou
R¹ et R² représentent avec l'atome d'azote sur lequel ils sont liés, un hétérocycle saturé ou insaturé ayant 3 à 6 membres cycliques, où l'hétérocycle peut contenir un autre atome d'azote, d'oxygène ou de soufre comme membre cyclique, et où l'hétérocycle peut être substitué jusqu'à trois fois par l'atome de fluor, de chlore, de brome, un radical alcoyle ayant 1 à 4 atomes de carbone et/ou un radical halogénoalcoyle ayant 1 à 4 atomes de carbone et 1 à 9 atomes de fluor et/ou de chlore,
R³ représente un radical hétérocyclyle saturé ou insaturé ayant 5 ou 6 membres cycliques et 1 à 4 hétéroatomes, comme l'oxygène, l'azote et/ou le soufre, où le radical hétérocyclyle peut être substitué un à quatre fois, de manière identique ou différente, par un atome de fluor, de chlore, de brome, un radical cyano, nitro, alcoyle, alcoxy, hydroximinoalcoyle ou alcoximinoalcoyle, ayant chaque fois, 1 à 3 atomes de carbone par reste alcoyle, un radical halogénoalcoyle ou halogénoalcoxy, ayant chaque fois, 1 à 3 atomes de carbone et 1 à 7 atomes d'halogène,
R⁴ représente l'atome d'hydrogène ou un radical alcoyle,
Hal représente un atome d'halogène, et
X représente un atome d'halogène, un radical cyano, nitro, alcoyle, alcényle le cas échéant substitué, un radical éventuellement substitué alcynyle, hydroxyalcoyle, alcoxyalcoyle, halogénoalcoyle, cycloalcoyle, formyle, thiocarbamoyle, alcoxycarbonyle, alcoylcarbonyle, hydroximinoalcoyle, alcoximinoalcoyle, alcoylthio, alcoylsulfinyle, alcoylsulfonyle ou alcoylaminocarbonyle.

2. Pyrazolopyrimidines de la formule (I) selon la revendication 1, dans lesquelles :
R¹ représente un radical alcoyle ayant 1 à 6 atomes de carbone, qui peut être substitué une à cinq fois, de manière identique ou différente, par un atome d'halogène, un radical cyano, hydroxy, alcoxy ayant 1 à 4 atomes de carbone et/ou cycloalcoyle ayant 3 à 6 atomes de carbone, ou
R¹ représente un radical alcényle ayant 2 à 6 atomes de carbone, qui peut être substitué une à trois fois, de manière identique ou différente, par un atome d'halogène, un radical cyano, hydroxy, alcoxy ayant 1 à 4 atomes de carbone et/ou cycloalcoyle ayant 3 à 6 atomes de carbone, ou
R¹ représente un radical alcynyle ayant 3 à 6 atomes de carbone, qui peut être substitué une à trois fois, de manière identique ou différente, par un atome d'halogène, un radical cyano, hydroxy, alcoxy ayant 1 à 4 atomes de carbone et/ou cycloalcoyle ayant 3 à 6 atomes de carbone, ou
R¹ représente un radical cycloalcoyle ayant 3 à 6 atomes de carbone, qui peut être substitué une à trois fois, de manière identique ou différente, par un atome d'halogène et/ou un radical alcoyle ayant 1 à 4 atomes de carbone, ou
R¹ représente un radical hétérocyclyle saturé ou insaturé ayant 5 ou 6 membres cycliques et 1 à 3 hétéroatomes, comme l'azote, l'oxygène et/ou le soufre, où le radical hétérocyclyle peut être substitué un ou deux fois, par un atome d'halogène, un radical alcoyle ayant 1 à 4 atomes de carbone, un radical cyano, nitro et/ou cycloalcoyle ayant 3 à 6 atomes de carbone,
R² représente l'atome d'hydrogène ou un radical alcoyle ayant 1 à 4 atomes de carbone, ou
R¹ et R² représentent avec l'atome d'azote sur lequel ils sont liés, un hétérocycle saturé ou insaturé ayant 3 à 6 membres cycliques, où l'hétérocycle peut contenir un autre atome d'azote, d'oxygène ou de soufre comme membre cyclique, et où l'hétérocycle peut être substitué jusqu'à trois fois par l'atome de fluor, de chlore, de brome, un radical alcoyle ayant 1 à 4 atomes de carbone et/ou halogénoalcoyle ayant 1 à 4 atomes de carbone et 1 à 9 atomes de fluor et/ou de chlore,
R³ représente un radical hétérocyclyle saturé ou insaturé ayant 5 ou 6 membres cycliques et 1 à 4 hétéroatomes, comme l'oxygène, l'azote et/ou le soufre, où le radical hétérocyclyle peut être substitué un à quatre fois, de manière identique ou différente, par un atome de fluor, de chlore, de brome, un radical cyano, nitro, alcoyle, alcoxy, hydroximinoalcoyle ou alcoximinoalcoyle, ayant chaque fois, 1 à 3 atomes de carbone par reste alcoyle, un radical halogénoalcoyle ou halogénoalcoxy, ayant chaque fois, 1 à 3 atomes de carbone et 1 à 7 atomes d'halogène,
R⁴ représente l'atome d'hydrogène ou un radical alcoyle ayant 1 à 4 atomes de carbone,
Hal représente l'atome de fluor, de chlore ou de brome, et
X représente le radical cyano, l'atome de fluor, de chlore, de brome, d'iode, un radical nitro, formyle, halogénoalcoyle ayant 1 à 6 atomes de carbone et 1 à 9 atomes de fluor, de chlore et/ou de brome, un radical alcoyle ayant 1 à 4 atomes de carbone, alcényle ayant 2 à 6 atomes de carbone, alcényle ayant 2 à 5 atomes de carbone dans la partie alcényle, substitué par le radical carboxy, méthoxycarbonyle ou éthoxycarbonyle, alcynyle ayant 2 à 6 atomes de carbone, alcynyle ayant 2 à 5 atomes de carbone, substitué par le radical carboxy, méthoxycarbonyle ou éthoxycarbonyle, un radical hydroxyalcoyle ayant 1 à 4 atomes de carbone, un radical alcoxyalcoyle ayant 1 à 4 atomes de carbone dans la partie alcoxy et 1 à 4 atomes de carbone dans la partie alcoyle, un radical cycloalcoyle ayant 3 à 6 atomes de carbone, le radical thiocarbamoyle, un radical alcoxycarbonyle ayant 1 à 4 atomes de carbone dans la partie alcoxy, un radical alcoylcarbonyle ayant 1 à 4 atomes de carbone dans la partie alcoyle, un radical hydroximinoalcoyle ayant 1 à 4 atomes de carbone dans la partie alcoyle, un radical alcoximinoalcoyle ayant 1 à 4 atomes de carbone dans la partie alcoxy et 1 à 4 atomes de carbone dans la partie alcoyle, un radical alcoylthio ayant 1 à 4 atomes de carbone, un radical alcoylsulfinyle ayant 1 à 4 atomes de carbone, un radical alcoylsulfonyle ayant 1 à 4 atomes de carbone, ou un radical alcoylaminocarbonyle ayant 1 à 4 atomes de carbone dans la partie alcoyle.

3. Pyrazolopyrimidines de la formule (I) selon la revendication 1 ou 2, dans lesquelles :
R¹ représente un reste de la formule : 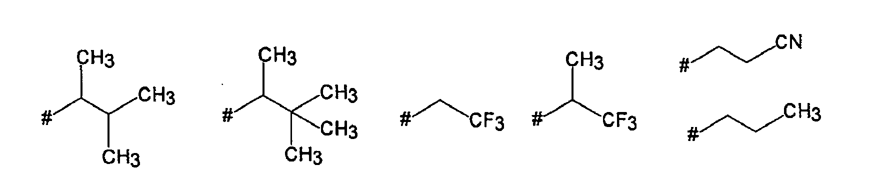 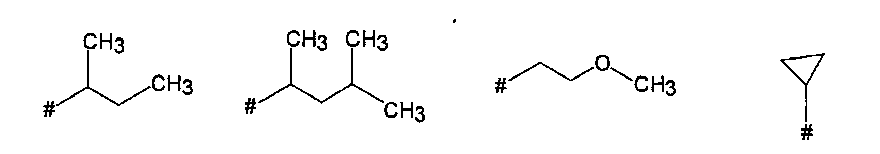 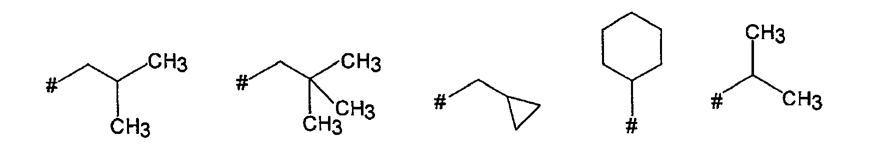 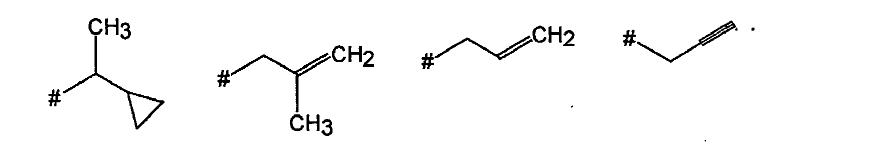 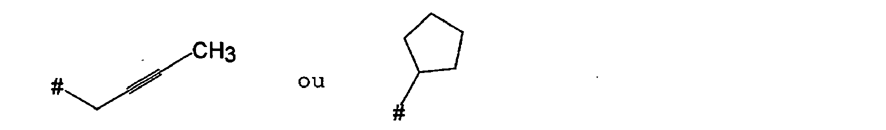
où # représente le site de liaison,
R² représente l'atome d'hydrogène, le radical méthyle, éthyle ou propyle, ou
R¹ et R² représentent avec l'atome d'azote sur lequel ils sont liés, un radical pyrrolidinyle, pipéridinyle, morpholinyle, thiomorpholinyle, pipérazinyle, 3,6-dihydro-1(2H)-pipéridinyle ou tétrahydro-1(2H)-pyridazinyle, où ces restes peuvent être substitués par 1 à 3 atomes de fluor, 1 à 3 radicaux méthyle et/ou trifluorométhyle, ou
R¹ et R² représentent avec l'atome d'azote sur lequel ils sont liés, un reste des formules : 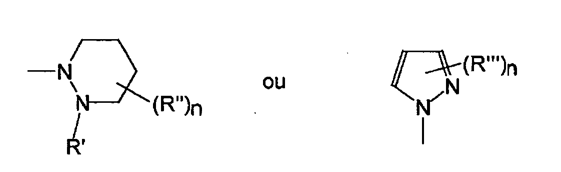
dans lesquelles :
R' représente l'atome d'hydrogène ou le radical méthyle,
R" représente le radical méthyle, éthyle, l'atome de fluor, de chlore ou le radical trifluorométhyle,
m représente les nombres 0, 1, 2 ou 3, où R" représente des restes identiques ou différents lorsque m est 2 ou 3,
R''' représente le radical méthyle, éthyle, l'atome de fluor, de chlore ou le radical trifluorométhyle, et
n représente les nombres 0, 1, 2 ou 3, où R"' représente des restes identiques ou différents lorsque n est 2 ou 3,
R³ représente un radical pyridyle, qui est relié en position 2 ou 4 et qui peut être substitué une à quatre fois, de manière identique ou différente par l'atome de fluor, de chlore, de brome, le radical cyano, nitro, méthyle, éthyle, méthoxy, méthylthio, hydroximinométhyle, hydroximinoéthyle, méthoximinométhyle, méthoximinoéthyle et/ou trifluorométhyle, ou
R³ représente un radical pyrimidyle, qui est relié en position 2 ou 4 et qui peut être substitué une à trois fois, de manière identique ou différente par l'atome de fluor, de chlore, de brome, le radical cyano, nitro, méthyle, éthyle, méthoxy, méthylthio, hydroximinométhyle, hydroximinoéthyle, méthoximinométhyle, méthoximinoéthyle et/ou trifluorométhyle, ou
R³ représente un radical thiényle, qui est relié en position 2 ou 3 et qui peut être substitué une à trois fois, de manière identique ou différente par l'atome de fluor, de chlore, de brome, le radical cyano, nitro, méthyle, éthyle, méthoxy, méthylthio, hydroximinométhyle, hydroximinoéthyle, méthoximinométhyle, méthoximinoéthyle et/ou trifluorométhyle, ou
R³ représente un radical thiazolyle, qui est relié en position 2, 4 ou 5 et qui peut être substitué une à deux fois, de manière identique ou différente par l'atome de fluor, de chlore, de brome, le radical cyano, nitro, méthyle, éthyle, méthoxy, méthylthio, hydroximinométhyle, hydroximinoéthyle, méthoximinométhyle, méthoximinoéthyle et/ou trifluorométhyle, ou
R⁴ représente l'atome d'hydrogène, le radical méthyle, éthyle, propyle ou isopropyle,
Hal représente l'atome de fluor ou de chlore,
X représente le radical cyano, l'atome de fluor, de chlore, de brome, d'iode, le radical nitro, formyle, trifluorométhyle, difluorométhyle, méthyle, éthyle, cyclopropyle, thiocarbamoyle, méthoxycarbonyle, méthylcarbonyle, éthylcarbonyle, hydroximinométhyle, méthoximinométhyle, méthylthio, méthylsulfinyle, méthylsulfonyle, méthylaminocarbonyle, hydroxyméthyle, hydroxyéthy-1-yle, méthoxyméthyle, éthoxyméthyle ou 1-méthoxyéthyle, ou
X représente un reste de la formule :

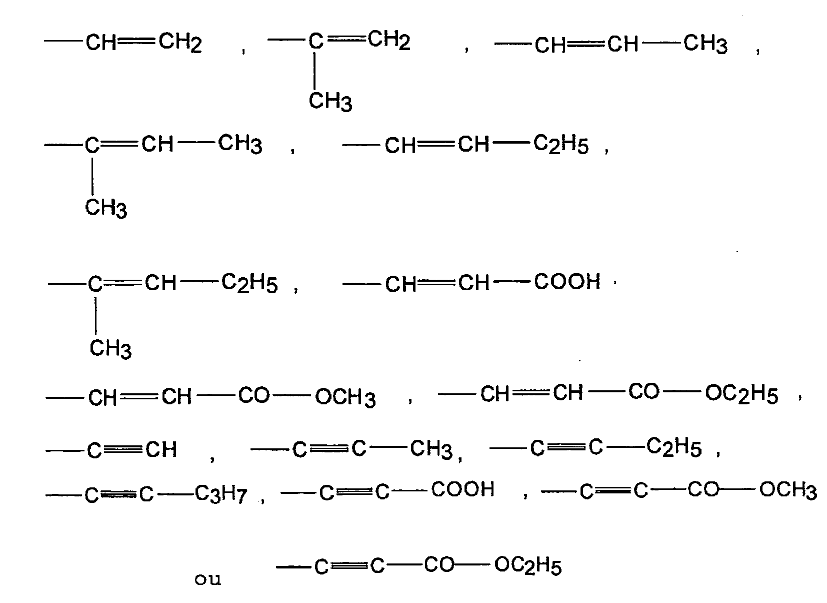

4. Procédé de préparation de pyrazolopyrimidines de la formule (I) selon la revendication 1, **caractérisé en ce que**
(a) on fait réagir des halogénopyrazolopyrimidines de la formule : 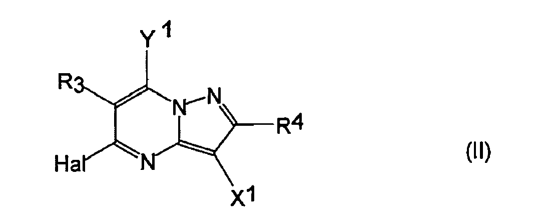 dans laquelle :
R³, R⁴ et Hal ont les significations données à la revendication 1,
X¹ représente un atome d'halogène, un radical cyano, nitro, alcoyle, halogénoalcoyle, cycloalcoyle, formyle, thiocarbamoyle, alcoxycarbonyle, alcoylcarbonyle, alcoylthio, alcoylsulfinyle, alcoylsulfonyle ou alcoylaminocarbonyle, et
Y¹ représente un atome d'halogène,
avec des amines de la formule : 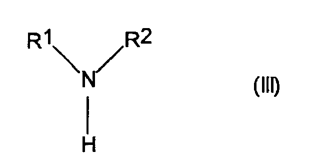
dans laquelle :
R¹ et R² ont les significations données à la revendication 1,
le cas échéant en présence d'un agent de dilution, le cas échéant en présence d'un accepteur d'acide et le cas échéant en présence d'un catalyseur,
ou
(b) on fait réagir des pyrazolopyrimidines de la formule : 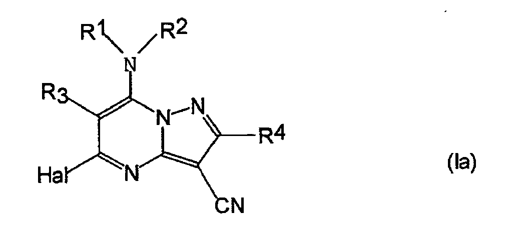 dans laquelle :
R¹, R², R³, R⁴ et Hal ont les significations données à la revendication 1,
soit
α) avec l'hydrure de diisobutyl-aluminium en présence d'une solution aqueuse de chlorure d'ammonium, ainsi qu'en présence d'un agent de dilution organique,
soit
β) avec des composés de Grignard de la formule :
R⁵ - Mg - X² (IV)
dans laquelle :
R⁵ représente un radical alcoyle, et
X² représente l'atome de chlore ou de brome,
en présence d'un agent de dilution et le cas échéant, en présence d'un catalyseur,
ou
c) on fait réagir des pyrazolopyrimidines de la formule : 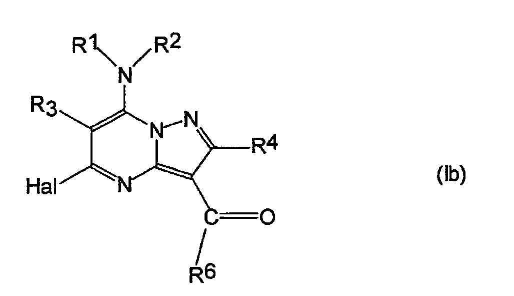 dans laquelle :
R¹, R², R³, R⁴ et Hal ont les significations données à la revendication 1, et
R⁶ représente l'atome d'hydrogène ou un radical alcoyle,
soit
α) avec des composés amino de la formule :
H₂N - OR⁷ (V)
dans laquelle :
R⁷ représente l'atome d'hydrogène ou un radical alcoyle,
en présence d'un agent de dilution et le cas échéant en présence d'un catalyseur, où les composés amino de la formule (V) peuvent également être mis en oeuvre sous forme de leur sel d'addition d'acide,
soit
β) avec des sels de triphénylphosphonium de la formule :
Ph₃P⊕ - CH₂ - R⁸ · Br ⁽⁻⁾ (VI)
dans laquelle :
Ph représente le radical phényle, et
R⁸ représente l'atome d'hydrogène ou un radical alcoyle le cas échéant substitué,
en présence d'une base, ainsi qu'en présence d'un agent de dilution,
soit
γ) avec l'hydrure de diisobutyl-aluminium en présence d'une solution aqueuse de chlorure d'ammonium, ainsi qu'en présence d'un agent de dilution organique,
ou avec le borohydrure de sodium en présence d'un agent de dilution,
et le cas échéant, les pyrazolopyrimidines ainsi formées, de la formule : 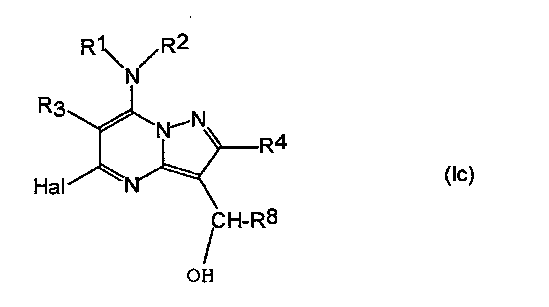
dans laquelle :
R¹, R², R³, R⁸ et Hal ont les significations données ci-dessus, et
sont mises à réagir avec des agents d'alcoylation de la formule :
R⁹ - X³ (VII)
dans laquelle :
R⁹ représente un radical alcoyle, et
X³ représente l'atome de chlore, de brome, d'iode ou le reste R⁹O-SO₂-O-,
le cas échéant en présence d'une base et en présence d'un agent de dilution,
ou
d) on fait réagir des pyrazolopyrimidines de la formule : 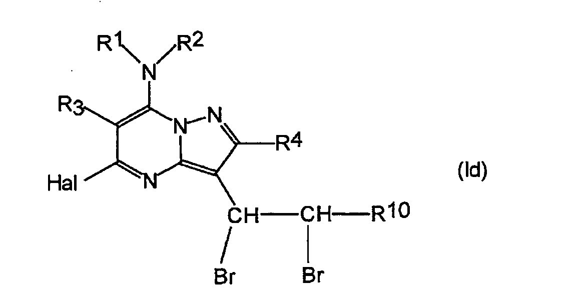 dans laquelle :
R¹, R², R³, R⁴ et Hal ont les significations données ci-dessus, et
R¹⁰ représente l'atome d'hydrogène ou un radical alcoyle le cas échéant substitué,
avec des bases fortes en présence d'un agent de dilution,
ou
e) on fait réagir des pyrazolopyrimidines de la formule : 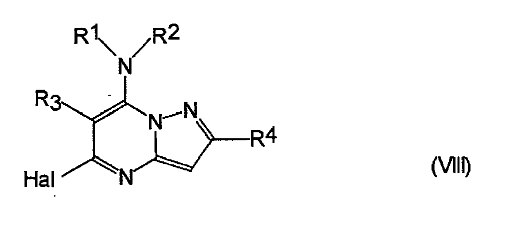 dans laquelle :
R¹, R², R³, R⁴ et Hal ont les significations données ci-dessus,
avec des dérivés acyle de la formule : 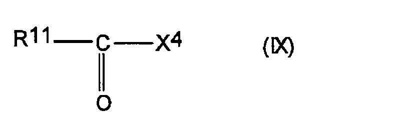 dans laquelle :
R¹¹ représente un radical alcoyle, et
x⁴ représente l'atome de chlore ou le reste -O-C(O)-R¹¹,
en présence d'un catalyseur et en présence d'un agent de dilution.

5. Agent pour lutter contre des microorganismes non souhaités, **caractérisé par** une teneur en au moins une pyrazolopyrimidine de la formule (I) selon une ou plusieurs des revendications 1 à 3, avec un délayant et/ou des substances tensioactives.

6. Utilisation des pyrazolopyrimidines de la formule (I) selon une ou plusieurs des revendications 1 à 3, pour la préparation d'un agent pour lutter contre des microorganismes non souhaités.

7. Procédé pour lutter contre des microorganismes non souhaités dans la protection des végétaux et dans la protection des matériaux, **caractérisé en ce que** l'on applique les pyrazolopyrimidines de la formule (I) selon une ou plusieurs des revendications 1 à 3, sur les microorganismes non souhaités et/ou leur biotope.

8. Procédé de préparation d'un agent pour lutter contre des microorganismes non souhaités, **caractérisé en ce que** l'on mélange des pyrazolopyrimidines de la formule (I) selon une ou plusieurs des revendications 1 à 3, avec des délayants et/ou des substances tensioactives.

9. Halogénopyrazolopyrimidines de la formule : 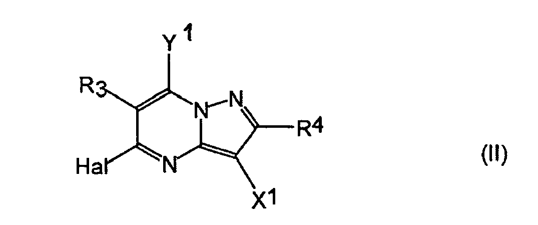 dans laquelle :
R³ représente un radical hétérocyclyle saturé ou insaturé ayant 5 ou 6 membres cycliques et 1 à 4 hétéroatomes, comme l'oxygène, l'azote et/ou le soufre, où le radical hétérocyclyle peut être substitué une à quatre fois, de manière identique ou différente, par un atome de fluor, de chlore, de brome, un radical cyano, nitro, alcoyle, alcoxy, hydroximinoalcoyle ou alcoximinoalcoyle, ayant chaque fois, 1 à 3 atomes de carbone par reste alcoyle, un radical halogénoalcoyle ou halogénoalcoxy, ayant chaque fois, 1 à 3 atomes de carbone et 1 à 7 atomes d'halogène,
R⁴ représente l'atome d'hydrogène ou un radical alcoyle,
Hal représente un atome d'halogène, et
X¹ représente un atome d'halogène, un radical cyano, nitro, alcoyle, halogénoalcoyle, cycloalcoyle, formyle, thiocarbamoyle, alcoxycarbonyle, alcoylcarbonyle, alcoylthio, alcoylsulfinyle, alcoylsulfonyle ou alcoylaminocarbonyle, et
Y¹ représente un atome d'halogène.

10. Procédé de préparation des halogénopyrazolopyrimidines de la formule (II) selon la revendication 9, **caractérisé en ce que**
f) on fait réagir des hydroxypyrazolopyrimidines de la formule : 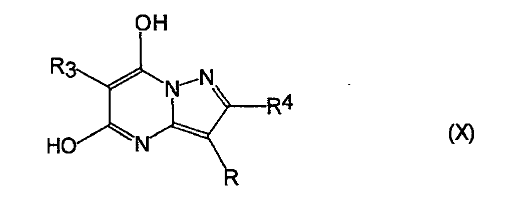 dans laquelle :
R³ et R⁴ ont les significations données à la revendication 9, et
R représente un atome d'halogène, un radical cyano, nitro, alcoyle, halogénoalcoyle, cycloalcoyle, thiocarbamoyle, alcoxycarbonyle, alcoylthio, alcoylsulfinyle, alcoylsulfonyle ou alcoylaminocarbonyle,
avec des agents d'halogénation, le cas échéant en présence d'un agent de dilution,
ou
g) on fait réagir des hydroxypyrazolopyrimidines de la formule : 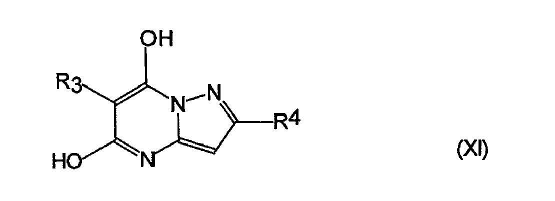 dans laquelle :
R³ et R⁴ ont les significations données à la revendication 9, et
avec l'oxychlorure de phosphore, en présence le diméthylformamide et le cas échéant, on fait réagir avec addition de pentachlorure de phosphore.

11. Hydroxypyrazolopyrimidines de la formule : 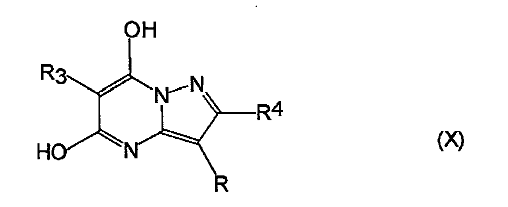 dans laquelle :
R³ représente un radical hétérocyclyle saturé ou insaturé ayant 5 ou 6 membres cycliques et 1 à 4 hétéroatomes, comme l'oxygène, l'azote et/ou le soufre, où le radical hétérocyclyle peut être substitué une à quatre fois, de manière identique ou différente, par un atome de fluor, de chlore, de brome, un radical cyano, nitro, alcoyle, alcoxy, hydroximinoalcoyle ou alcoximinoalcoyle, ayant chaque fois, 1 à 3 atomes de carbone par reste alcoyle, un radical halogénoalcoyle ou halogénoalcoxy, ayant chaque fois, 1 à 3 atomes de carbone et 1 à 7 atomes d'halogène,
R⁴ représente l'atome d'hydrogène ou un radical alcoyle,
R représente un atome d'halogène, un radical cyano, nitro, alcoyle, halogénoalcoyle, cycloalcoyle, thiocarbamoyle, alcoxycarbonyle, alcoylthio, alcoylsulfinyle, alcoylsulfonyle ou alcoylaminocarbonyle.

12. Procédé de préparation des hydroxypyrazolopyrimidines de la formule (X) selon la revendication 11, **caractérisé en ce que**
(h) on fait réagir des esters hétérocyclylmaloniques de la formule : 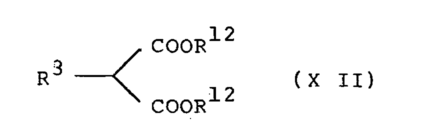 dans laquelle :
R³ a la signification donnée à la revendication 11, et
R¹² représente un radical alcoyle ayant 1 à 4 atomes de carbone,
avec des aminopyrazoles de la formule : 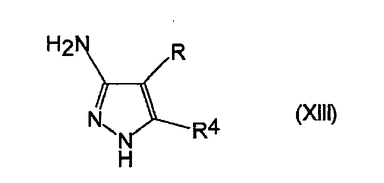 dans laquelle :
R⁴ et R ont les significations données à la revendication 11,
le cas échéant en présence d'un agent de dilution et le cas échéant, en présence d'un agent liant les acides.
